# EUROPEAN PATENT APPLICATION

(11) **EP 2 631 240 A1**
(43) Date of publication of application: **28.08.2013**
(21) Application number: 13168458.1
(22) Date of filing: 20.05.2010
(51) Int. Cl.: C07H 21/00, G01N 33/48, C12P 19/04, C12Q 1/70

(54) **Optimized probes and primers and methods of using same for the detection, screening, quantitation, isolation and sequencing of Cytomegalovirus and Epstein-Barr virus**

(30) Priority: 22.05.2009 US 180671 P
(62) Divisional of application: 10778374.8
(71) Applicant: Intelligent Medical Devices, Inc., Cambridge, Massachusetts 02139 (US)
(72) Inventor: Slater, Damien, Roslindale, MA Massachusetts 60060 (US); Hully, James R, Mundelein, IL Illinois 02131 (US); Dolinger, David, Medford, MA Massachusetts 02053 (US); Jacobs, Alice A, Cambridge, MA Massachusetts 02139 (US)
(74) Representative: Murphy, Colm Damien

(57) **Abstract**

The invention is directed to primers and probes useful for detecting, screening, quantitating, isolating and sequencing EBV viral strains, and methods of using the described primers and probes.

SEQUENCE LISTING

<110> INTELLIGENT MEDICAL DEVICES, INC.

<120> OPTIMIZED PROBES AND PRIMERS AND METHODS OF USING SAME FOR THE
      DETECTION, SCREENING, QUANTITATION, ISOLATION AND SEQUENCING OF
      CYTOMEGALOVIRUS AND EPSTEIN-BARR VIRUS

<130> 1156-0204

<140> EP10778374.8
<141> 2010-05-20

<150> 61/180,671
<151> 2009-05-22

<160> 196

<170> PatentIn version 3.5

<210> 1
<211> 18
<212> DNA
<213> Artificial Sequence

<220>
<223> Description of Artificial Sequence: Synthetic
      primer

<400> 1
gtagacctgt cgttgtat                                                    18


<210> 2
<211> 17
<212> DNA
<213> Artificial Sequence

<220>
<223> Description of Artificial Sequence: Synthetic
      primer

<400> 2
ataaccttgt gggttgt                                                     17


<210> 3
<211> 15
<212> DNA
<213> Artificial Sequence

<220>
<223> Description of Artificial Sequence: Synthetic
      primer

<400> 3
agccgttcgt gtgat                                                       15


<210> 4
<211> 17
<212> DNA
<213> Artificial Sequence

<220>
<223> Description of Artificial Sequence: Synthetic
      primer

<400> 4
agacccgaga cagtaat                                                    17


<210> 5
<211> 18
<212> DNA
<213> Artificial Sequence

<220>
<223> Description of Artificial Sequence: Synthetic
      primer

<400> 5
gaggtgagag tccatttg                                                   18


<210> 6
<211> 17
<212> DNA
<213> Artificial Sequence

<220>
<223> Description of Artificial Sequence: Synthetic
      primer

<400> 6
ggtgagagtc catttgc                                                    17


<210> 7
<211> 17
<212> DNA
<213> Artificial Sequence

<220>
<223> Description of Artificial Sequence: Synthetic
      primer

<400> 7
aaaaggcagt cactgta                                                    17


<210> 8
<211> 17
<212> DNA
<213> Artificial Sequence

<220>
<223> Description of Artificial Sequence: Synthetic
      primer

<400> 8
aaaggcagtc actgtaa                                                    17


<210> 9
<211> 17

<212> DNA
<213> Artificial Sequence

<220>
<223> Description of Artificial Sequence: Synthetic
      primer

<400> 9
ggcagtcact gtaacat                                                    17


<210> 10
<211> 17
<212> DNA
<213> Artificial Sequence

<220>
<223> Description of Artificial Sequence: Synthetic
      primer

<400> 10
gtttgcgtcc tcagaag                                                    17


<210> 11
<211> 16
<212> DNA
<213> Artificial Sequence

<220>
<223> Description of Artificial Sequence: Synthetic
      primer

<400> 11
tgcgtcctca gaagtg                                                     16


<210> 12
<211> 17
<212> DNA
<213> Artificial Sequence

<220>
<223> Description of Artificial Sequence: Synthetic
      primer

<400> 12
attggcactg aataggt                                                    17


<210> 13
<211> 19
<212> DNA
<213> Artificial Sequence

<220>
<223> Description of Artificial Sequence: Synthetic
      primer

<400> 13
cactgaatag gtagcattg                                                  19

<210> 14
<211> 15
<212> DNA
<213> Artificial Sequence

<220>
<223> Description of Artificial Sequence: Synthetic
      primer

<400> 14
acgcaggctg tatgc                                                        15


<210> 15
<211> 17
<212> DNA
<213> Artificial Sequence

<220>
<223> Description of Artificial Sequence: Synthetic
      primer

<400> 15
accctgtacc aggtatt                                                      17


<210> 16
<211> 16
<212> DNA
<213> Artificial Sequence

<220>
<223> Description of Artificial Sequence: Synthetic
      primer

<400> 16
cacgggactc gttgat                                                       16


<210> 17
<211> 16
<212> DNA
<213> Artificial Sequence

<220>
<223> Description of Artificial Sequence: Synthetic
      primer

<400> 17
acgggactcg ttgatg                                                       16


<210> 18
<211> 16
<212> DNA
<213> Artificial Sequence

<220>
<223> Description of Artificial Sequence: Synthetic
      primer

<400> 18
cgggactcgt tgatgt                                                       16

<210> 19
<211> 15
<212> DNA
<213> Artificial Sequence

<220>
<223> Description of Artificial Sequence: Synthetic
      primer

<400> 19
tgtctccggg cagaa                                                        15


<210> 20
<211> 16
<212> DNA
<213> Artificial Sequence

<220>
<223> Description of Artificial Sequence: Synthetic
      primer

<400> 20
tgaaagtgct ggctat                                                       16


<210> 21
<211> 17
<212> DNA
<213> Artificial Sequence

<220>
<223> Description of Artificial Sequence: Synthetic
      primer

<400> 21
tgaaagtgct ggctatc                                                      17


<210> 22
<211> 18
<212> DNA
<213> Artificial Sequence

<220>
<223> Description of Artificial Sequence: Synthetic
      primer

<400> 22
cttcttagac cgcatagt                                                     18


<210> 23
<211> 17
<212> DNA
<213> Artificial Sequence

<220>
<223> Description of Artificial Sequence: Synthetic
      primer

<400> 23
tcttctacat cgtgaca                                                    17


<210> 24
<211> 15
<212> DNA
<213> Artificial Sequence

<220>
<223> Description of Artificial Sequence: Synthetic
      primer

<400> 24
ggccgcagtt tgcta                                                      15


<210> 25
<211> 17
<212> DNA
<213> Artificial Sequence

<220>
<223> Description of Artificial Sequence: Synthetic
      primer

<400> 25
ccgcagtttg ctagaag                                                    17


<210> 26
<211> 17
<212> DNA
<213> Artificial Sequence

<220>
<223> Description of Artificial Sequence: Synthetic
      primer

<400> 26
tcttggcgta gttgaat                                                    17


<210> 27
<211> 15
<212> DNA
<213> Artificial Sequence

<220>
<223> Description of Artificial Sequence: Synthetic
      primer

<400> 27
gttcccgacg aggtt                                                      15


<210> 28
<211> 18
<212> DNA
<213> Artificial Sequence

<220>
<223> Description of Artificial Sequence: Synthetic

primer

<400> 28
gaaacatgat cctgagaa                                                    18


<210> 29
<211> 18
<212> DNA
<213> Artificial Sequence

<220>
<223> Description of Artificial Sequence: Synthetic
      primer

<400> 29
aaacatgatc ctgagaac                                                    18


<210> 30
<211> 17
<212> DNA
<213> Artificial Sequence

<220>
<223> Description of Artificial Sequence: Synthetic
      primer

<400> 30
gatcctgaga acgtcat                                                     17


<210> 31
<211> 16
<212> DNA
<213> Artificial Sequence

<220>
<223> Description of Artificial Sequence: Synthetic
      primer

<400> 31
agaaaggatg gcaact                                                      16


<210> 32
<211> 16
<212> DNA
<213> Artificial Sequence

<220>
<223> Description of Artificial Sequence: Synthetic
      primer

<400> 32
cgaactggtg gacaca                                                      16


<210> 33
<211> 16
<212> DNA
<213> Artificial Sequence

<220>
<223> Description of Artificial Sequence: Synthetic
      primer

<400> 33
cgtcgttcgt gaatcc                                                       16


<210> 34
<211> 16
<212> DNA
<213> Artificial Sequence

<220>
<223> Description of Artificial Sequence: Synthetic
      primer

<400> 34
actggtggac acatga                                                       16


<210> 35
<211> 17
<212> DNA
<213> Artificial Sequence

<220>
<223> Description of Artificial Sequence: Synthetic
      primer

<400> 35
cggtgtacga tgaactg                                                      17


<210> 36
<211> 17
<212> DNA
<213> Artificial Sequence

<220>
<223> Description of Artificial Sequence: Synthetic
      primer

<400> 36
ggtgtacgat gaactga                                                      17


<210> 37
<211> 22
<212> DNA
<213> Artificial Sequence

<220>
<223> Description of Artificial Sequence: Synthetic
      probe

<400> 37
ccaccacctc ccctaccttg aa                                                22


<210> 38
<211> 21
<212> DNA

<213> Artificial Sequence

<220>
<223> Description of Artificial Sequence: Synthetic
      probe

<400> 38
acgaacggct accaatgcca c                                                21


<210> 39
<211> 24
<212> DNA
<213> Artificial Sequence

<220>
<223> Description of Artificial Sequence: Synthetic
      probe

<400> 39
attatcacac gaacggctac caat                                             24


<210> 40
<211> 22
<212> DNA
<213> Artificial Sequence

<220>
<223> Description of Artificial Sequence: Synthetic
      probe

<400> 40
cgtgccaaga cccgagacag ta                                               22


<210> 41
<211> 21
<212> DNA
<213> Artificial Sequence

<220>
<223> Description of Artificial Sequence: Synthetic
      probe

<400> 41
ttgctcgcaa atgcaccaga a                                                21


<210> 42
<211> 22
<212> DNA
<213> Artificial Sequence

<220>
<223> Description of Artificial Sequence: Synthetic
      probe

<400> 42
tgctcgcaaa tgcaccagaa at                                               22


<210> 43

<211> 23
<212> DNA
<213> Artificial Sequence

<220>
<223> Description of Artificial Sequence: Synthetic
      probe

<400> 43
tgctcgcaaa tgcaccagaa ata                                              23


<210> 44
<211> 23
<212> DNA
<213> Artificial Sequence

<220>
<223> Description of Artificial Sequence: Synthetic
      probe

<400> 44
tggccaaaca acactgaaac tga                                              23


<210> 45
<211> 24
<212> DNA
<213> Artificial Sequence

<220>
<223> Description of Artificial Sequence: Synthetic
      probe

<400> 45
ctggccaaac aacactgaaa ctga                                             24


<210> 46
<211> 23
<212> DNA
<213> Artificial Sequence

<220>
<223> Description of Artificial Sequence: Synthetic
      probe

<400> 46
cctggccaaa caacactgaa act                                              23


<210> 47
<211> 22
<212> DNA
<213> Artificial Sequence

<220>
<223> Description of Artificial Sequence: Synthetic
      probe

<400> 47
tttgcgtcct cagaagtgac ca                                               22

<210> 48
<211> 23
<212> DNA
<213> Artificial Sequence

<220>
<223> Description of Artificial Sequence: Synthetic
      probe

<400> 48
tttgcgtcct cagaagtgac cat                                               23


<210> 49
<211> 22
<212> DNA
<213> Artificial Sequence

<220>
<223> Description of Artificial Sequence: Synthetic
      probe

<400> 49
ttgcgtcctc agaagtgacc at                                                22


<210> 50
<211> 23
<212> DNA
<213> Artificial Sequence

<220>
<223> Description of Artificial Sequence: Synthetic
      probe

<400> 50
tgcgtcctca gaagtgacca ttg                                               23


<210> 51
<211> 23
<212> DNA
<213> Artificial Sequence

<220>
<223> Description of Artificial Sequence: Synthetic
      probe

<400> 51
cgtcctcaga agtgaccatt ggc                                               23


<210> 52
<211> 21
<212> DNA
<213> Artificial Sequence

<220>
<223> Description of Artificial Sequence: Synthetic
      probe

<400> 52

tgaggacgca aactcgccaa a                                                21

<210> 53
<211> 22
<212> DNA
<213> Artificial Sequence

<220>
<223> Description of Artificial Sequence: Synthetic
      probe

<400> 53
cttctgagga cgcaaactcg cc                                               22

<210> 54
<211> 22
<212> DNA
<213> Artificial Sequence

<220>
<223> Description of Artificial Sequence: Synthetic
      probe

<400> 54
acttctgagg acgcaaactc gc                                               22

<210> 55
<211> 23
<212> DNA
<213> Artificial Sequence

<220>
<223> Description of Artificial Sequence: Synthetic
      probe

<400> 55
cacttctgag gacgcaaact cgc                                              23

<210> 56
<211> 23
<212> DNA
<213> Artificial Sequence

<220>
<223> Description of Artificial Sequence: Synthetic
      probe

<400> 56
tggtcacttc tgaggacgca aac                                              23

<210> 57
<211> 23
<212> DNA
<213> Artificial Sequence

<220>
<223> Description of Artificial Sequence: Synthetic
      probe

<400> 57
ccaatggtca cttctgagga cgc                                              23

<210> 58
<211> 24
<212> DNA
<213> Artificial Sequence

<220>
<223> Description of Artificial Sequence: Synthetic
      probe

<400> 58
acaatgctac ctattcagtg ccaa                                             24

<210> 59
<211> 21
<212> DNA
<213> Artificial Sequence

<220>
<223> Description of Artificial Sequence: Synthetic
      probe

<400> 59
aggacatgcc gacaaacacc a                                                21

<210> 60
<211> 22
<212> DNA
<213> Artificial Sequence

<220>
<223> Description of Artificial Sequence: Synthetic
      probe

<400> 60
aggacatgcc gacaaacacc ac                                               22

<210> 61
<211> 22
<212> DNA
<213> Artificial Sequence

<220>
<223> Description of Artificial Sequence: Synthetic
      probe

<400> 61
caggacatgc cgacaaacac ca                                               22

<210> 62
<211> 23
<212> DNA
<213> Artificial Sequence

<220>

<223> Description of Artificial Sequence: Synthetic
      probe

<400> 62
cggcggaatt ctcacatcaa cga                                              23


<210> 63
<211> 21
<212> DNA
<213> Artificial Sequence

<220>
<223> Description of Artificial Sequence: Synthetic
      probe

<400> 63
ccagcggcgg aattctcaca t                                                21


<210> 64
<211> 20
<212> DNA
<213> Artificial Sequence

<220>
<223> Description of Artificial Sequence: Synthetic
      probe

<400> 64
tcccagcggc ggaattctca                                                  20


<210> 65
<211> 23
<212> DNA
<213> Artificial Sequence

<220>
<223> Description of Artificial Sequence: Synthetic
      probe

<400> 65
aacatggctc tcgtatccac tgc                                              23


<210> 66
<211> 24
<212> DNA
<213> Artificial Sequence

<220>
<223> Description of Artificial Sequence: Synthetic
      probe

<400> 66
ctcgtatcca ctgcaggtga tgtt                                             24


<210> 67
<211> 23
<212> DNA
<213> Artificial Sequence

<220>
<223> Description of Artificial Sequence: Synthetic
      probe

<400> 67
aacatcacct gcagtggata cga                                               23


<210> 68
<211> 24
<212> DNA
<213> Artificial Sequence

<220>
<223> Description of Artificial Sequence: Synthetic
      probe

<400> 68
ttgtgaaatt tcgccatcct ccat                                              24


<210> 69
<211> 23
<212> DNA
<213> Artificial Sequence

<220>
<223> Description of Artificial Sequence: Synthetic
      probe

<400> 69
tgtgaaattt cgccatcctc cat                                               23


<210> 70
<211> 24
<212> DNA
<213> Artificial Sequence

<220>
<223> Description of Artificial Sequence: Synthetic
      probe

<400> 70
tgtgaaattt cgccatcctc cata                                              24


<210> 71
<211> 22
<212> DNA
<213> Artificial Sequence

<220>
<223> Description of Artificial Sequence: Synthetic
      probe

<400> 71
agagccaatt ctcctgcacc aa                                                22


<210> 72
<211> 23

<212> DNA
<213> Artificial Sequence

<220>
<223> Description of Artificial Sequence: Synthetic
      probe

<400> 72
agagccaatt ctcctgcacc aag                                               23


<210> 73
<211> 22
<212> DNA
<213> Artificial Sequence

<220>
<223> Description of Artificial Sequence: Synthetic
      probe

<400> 73
agccaattct cctgcaccaa gg                                                22


<210> 74
<211> 23
<212> DNA
<213> Artificial Sequence

<220>
<223> Description of Artificial Sequence: Synthetic
      probe

<400> 74
tcttctacat cgtgacaggc acc                                               23


<210> 75
<211> 24
<212> DNA
<213> Artificial Sequence

<220>
<223> Description of Artificial Sequence: Synthetic
      probe

<400> 75
cttctacatc gtgacaggca cctc                                              24


<210> 76
<211> 23
<212> DNA
<213> Artificial Sequence

<220>
<223> Description of Artificial Sequence: Synthetic
      probe

<400> 76
ttctacatcg tgacaggcac ctc                                               23

<210> 77
<211> 23
<212> DNA
<213> Artificial Sequence

<220>
<223> Description of Artificial Sequence: Synthetic
      probe

<400> 77
tctacatcgt gacaggcacc tcc                                              23


<210> 78
<211> 22
<212> DNA
<213> Artificial Sequence

<220>
<223> Description of Artificial Sequence: Synthetic
      probe

<400> 78
tacatcgtga caggcacctc ca                                               22


<210> 79
<211> 21
<212> DNA
<213> Artificial Sequence

<220>
<223> Description of Artificial Sequence: Synthetic
      probe

<400> 79
acatcgtgac aggcacctcc a                                                21


<210> 80
<211> 21
<212> DNA
<213> Artificial Sequence

<220>
<223> Description of Artificial Sequence: Synthetic
      probe

<400> 80
catcgtgaca ggcacctcca t                                                21


<210> 81
<211> 22
<212> DNA
<213> Artificial Sequence

<220>
<223> Description of Artificial Sequence: Synthetic
      probe

<400> 81
catcgtgaca ggcacctcca tt                                               22

<210> 82
<211> 24
<212> DNA
<213> Artificial Sequence

<220>
<223> Description of Artificial Sequence: Synthetic
      probe

<400> 82
ctccattttc agcttcatag cggt                                              24


<210> 83
<211> 23
<212> DNA
<213> Artificial Sequence

<220>
<223> Description of Artificial Sequence: Synthetic
      probe

<400> 83
tccattttca gcttcatagc ggt                                               23


<210> 84
<211> 25
<212> DNA
<213> Artificial Sequence

<220>
<223> Description of Artificial Sequence: Synthetic
      probe

<400> 84
ccattttcag cttcatagcg gtact                                             25


<210> 85
<211> 23
<212> DNA
<213> Artificial Sequence

<220>
<223> Description of Artificial Sequence: Synthetic
      probe

<400> 85
ccacctatct acctcccagc act                                               23


<210> 86
<211> 24
<212> DNA
<213> Artificial Sequence

<220>
<223> Description of Artificial Sequence: Synthetic
      probe

<400> 86
ccacctatct acctcccagc actt                                              24

<210> 87
<211> 24
<212> DNA
<213> Artificial Sequence

<220>
<223> Description of Artificial Sequence: Synthetic
      probe

<400> 87
aatgcgacca cctatctacc tccc                                              24

<210> 88
<211> 24
<212> DNA
<213> Artificial Sequence

<220>
<223> Description of Artificial Sequence: Synthetic
      probe

<400> 88
acaatgcgac cacctatcta cctc                                              24

<210> 89
<211> 24
<212> DNA
<213> Artificial Sequence

<220>
<223> Description of Artificial Sequence: Synthetic
      probe

<400> 89
caagtacaga gcccaccaca gatt                                              24

<210> 90
<211> 22
<212> DNA
<213> Artificial Sequence

<220>
<223> Description of Artificial Sequence: Synthetic
      probe

<400> 90
cggacaagta cagagcccac ca                                                22

<210> 91
<211> 20
<212> DNA
<213> Artificial Sequence

<220>
<223> Description of Artificial Sequence: Synthetic

probe

<400> 91
tcagatcccc gccggatttt                                                   20


<210> 92
<211> 21
<212> DNA
<213> Artificial Sequence

<220>
<223> Description of Artificial Sequence: Synthetic
      probe

<400> 92
cagatccccg ccggattttg t                                                 21


<210> 93
<211> 22
<212> DNA
<213> Artificial Sequence

<220>
<223> Description of Artificial Sequence: Synthetic
      probe

<400> 93
cagatccccg ccggattttg tt                                                22


<210> 94
<211> 20
<212> DNA
<213> Artificial Sequence

<220>
<223> Description of Artificial Sequence: Synthetic
      probe

<400> 94
agatccccgc cggattttgt                                                   20


<210> 95
<211> 21
<212> DNA
<213> Artificial Sequence

<220>
<223> Description of Artificial Sequence: Synthetic
      probe

<400> 95
agatccccgc cggattttgt t                                                 21


<210> 96
<211> 25
<212> DNA
<213> Artificial Sequence

<220>
<223> Description of Artificial Sequence: Synthetic
      probe

<400> 96
ccaagttgcc atcctttctg aaata                                             25


<210> 97
<211> 22
<212> DNA
<213> Artificial Sequence

<220>
<223> Description of Artificial Sequence: Synthetic
      probe

<400> 97
tccaagttgc catcctttct ga                                                22


<210> 98
<211> 25
<212> DNA
<213> Artificial Sequence

<220>
<223> Description of Artificial Sequence: Synthetic
      probe

<400> 98
cgtcgttcgt gaatcccaat tatgg                                             25


<210> 99
<211> 21
<212> DNA
<213> Artificial Sequence

<220>
<223> Description of Artificial Sequence: Synthetic
      probe

<400> 99
agaggctcgc ctttgaccat c                                                 21


<210> 100
<211> 22
<212> DNA
<213> Artificial Sequence

<220>
<223> Description of Artificial Sequence: Synthetic
      probe

<400> 100
aggcgagcct ctcccataat tg                                                22


<210> 101
<211> 21
<212> DNA

<213> Artificial Sequence

<220>
<223> Description of Artificial Sequence: Synthetic
      probe

<400> 101
acacaggtga caccagcctc t                                                21


<210> 102
<211> 22
<212> DNA
<213> Artificial Sequence

<220>
<223> Description of Artificial Sequence: Synthetic
      probe

<400> 102
acacaggtga caccagcctc ta                                               22


<210> 103
<211> 22
<212> DNA
<213> Artificial Sequence

<220>
<223> Description of Artificial Sequence: Synthetic
      probe

<400> 103
accgcctcgg aaatggaaga ac                                               22


<210> 104
<211> 22
<212> DNA
<213> Artificial Sequence

<220>
<223> Description of Artificial Sequence: Synthetic
      probe

<400> 104
ccgcctcgga aatggaagaa cc                                               22


<210> 105
<211> 22
<212> DNA
<213> Artificial Sequence

<220>
<223> Description of Artificial Sequence: Synthetic
      probe

<400> 105
cgcctcggaa atggaagaac ct                                               22


<210> 106

<211> 23
<212> DNA
<213> Artificial Sequence

<220>
<223> Description of Artificial Sequence: Synthetic
      probe

<400> 106
cgcctcggaa atggaagaac ctt                                              23


<210> 107
<211> 21
<212> DNA
<213> Artificial Sequence

<220>
<223> Description of Artificial Sequence: Synthetic
      probe

<400> 107
ttcttccatt tccgaggcgg t                                                21


<210> 108
<211> 22
<212> DNA
<213> Artificial Sequence

<220>
<223> Description of Artificial Sequence: Synthetic
      probe

<400> 108
aggttcttcc atttccgagg cg                                               22


<210> 109
<211> 19
<212> DNA
<213> Artificial Sequence

<220>
<223> Description of Artificial Sequence: Synthetic
      primer

<400> 109
cccacaaggt tatattctc                                                   19


<210> 110
<211> 17
<212> DNA
<213> Artificial Sequence

<220>
<223> Description of Artificial Sequence: Synthetic
      primer

<400> 110
ttactgtctc gggtctt                                                     17

<210> 111
<211> 17
<212> DNA
<213> Artificial Sequence

<220>
<223> Description of Artificial Sequence: Synthetic
      primer

<400> 111
gcaatcggac atttgac                                                    17


<210> 112
<211> 17
<212> DNA
<213> Artificial Sequence

<220>
<223> Description of Artificial Sequence: Synthetic
      primer

<400> 112
gagcaatcgg acatttg                                                    17


<210> 113
<211> 16
<212> DNA
<213> Artificial Sequence

<220>
<223> Description of Artificial Sequence: Synthetic
      primer

<400> 113
cgagcaatcg gacatt                                                     16


<210> 114
<211> 16
<212> DNA
<213> Artificial Sequence

<220>
<223> Description of Artificial Sequence: Synthetic
      primer

<400> 114
gcatttgcga gcaatc                                                     16


<210> 115
<211> 17
<212> DNA
<213> Artificial Sequence

<220>
<223> Description of Artificial Sequence: Synthetic
      primer

<400> 115

cttcgggttg tgaaaat                                                    17

<210> 116
<211> 17
<212> DNA
<213> Artificial Sequence

<220>
<223> Description of Artificial Sequence: Synthetic
      primer

<400> 116
gtgcaaatgg actctca                                                    17

<210> 117
<211> 16
<212> DNA
<213> Artificial Sequence

<220>
<223> Description of Artificial Sequence: Synthetic
      primer

<400> 117
acagtgactg cctttt                                                     16

<210> 118
<211> 17
<212> DNA
<213> Artificial Sequence

<220>
<223> Description of Artificial Sequence: Synthetic
      primer

<400> 118
tacagtgact gcctttt                                                    17

<210> 119
<211> 17
<212> DNA
<213> Artificial Sequence

<220>
<223> Description of Artificial Sequence: Synthetic
      primer

<400> 119
ttacagtgac tgccttt                                                    17

<210> 120
<211> 16
<212> DNA
<213> Artificial Sequence

<220>
<223> Description of Artificial Sequence: Synthetic
      primer

<400> 120
gtgccaatgg tcactt                                                        16


<210> 121
<211> 16
<212> DNA
<213> Artificial Sequence

<220>
<223> Description of Artificial Sequence: Synthetic
      primer

<400> 121
attcagtgcc aatggt                                                        16


<210> 122
<211> 17
<212> DNA
<213> Artificial Sequence

<220>
<223> Description of Artificial Sequence: Synthetic
      primer

<400> 122
acctattcag tgccaat                                                       17


<210> 123
<211> 19
<212> DNA
<213> Artificial Sequence

<220>
<223> Description of Artificial Sequence: Synthetic
      primer

<400> 123
gacaatgcta cctattcag                                                     19


<210> 124
<211> 19
<212> DNA
<213> Artificial Sequence

<220>
<223> Description of Artificial Sequence: Synthetic
      primer

<400> 124
gtgacaatgc tacctattc                                                     19


<210> 125
<211> 16
<212> DNA
<213> Artificial Sequence

<220>

<223> Description of Artificial Sequence: Synthetic
      primer

<400> 125
atgtgggtga caatgc                                                        16


<210> 126
<211> 18
<212> DNA
<213> Artificial Sequence

<220>
<223> Description of Artificial Sequence: Synthetic
      primer

<400> 126
catatgtggg tgacaatg                                                      18


<210> 127
<211> 17
<212> DNA
<213> Artificial Sequence

<220>
<223> Description of Artificial Sequence: Synthetic
      primer

<400> 127
acatatgtgg gtgacaa                                                       17


<210> 128
<211> 17
<212> DNA
<213> Artificial Sequence

<220>
<223> Description of Artificial Sequence: Synthetic
      primer

<400> 128
caccacagac atcacat                                                       17


<210> 129
<211> 16
<212> DNA
<213> Artificial Sequence

<220>
<223> Description of Artificial Sequence: Synthetic
      primer

<400> 129
acaaacacca cagaca                                                        16


<210> 130
<211> 17
<212> DNA
<213> Artificial Sequence

<220>
<223> Description of Artificial Sequence: Synthetic
      primer

<400> 130
atgagattcc agcttca                                                      17


<210> 131
<211> 17
<212> DNA
<213> Artificial Sequence

<220>
<223> Description of Artificial Sequence: Synthetic
      primer

<400> 131
tgatgagatt ccagctt                                                      17


<210> 132
<211> 17
<212> DNA
<213> Artificial Sequence

<220>
<223> Description of Artificial Sequence: Synthetic
      primer

<400> 132
atacgagagc catgttc                                                      17


<210> 133
<211> 17
<212> DNA
<213> Artificial Sequence

<220>
<223> Description of Artificial Sequence: Synthetic
      primer

<400> 133
gatacgagag ccatgtt                                                      17


<210> 134
<211> 16
<212> DNA
<213> Artificial Sequence

<220>
<223> Description of Artificial Sequence: Synthetic
      primer

<400> 134
acctgcagtg gatacg                                                       16


<210> 135
<211> 15

<212> DNA
<213> Artificial Sequence

<220>
<223> Description of Artificial Sequence: Synthetic
      primer

<400> 135
gcccggagac aacaa                                                        15


<210> 136
<211> 15
<212> DNA
<213> Artificial Sequence

<220>
<223> Description of Artificial Sequence: Synthetic
      primer

<400> 136
tctgcccgga gacaa                                                        15


<210> 137
<211> 18
<212> DNA
<213> Artificial Sequence

<220>
<223> Description of Artificial Sequence: Synthetic
      primer

<400> 137
gctcggtaat gagataga                                                     18


<210> 138
<211> 18
<212> DNA
<213> Artificial Sequence

<220>
<223> Description of Artificial Sequence: Synthetic
      primer

<400> 138
tgctcggtaa tgagatag                                                     18


<210> 139
<211> 17
<212> DNA
<213> Artificial Sequence

<220>
<223> Description of Artificial Sequence: Synthetic
      primer

<400> 139
aatgctcggt aatgaga                                                      17

<210> 140
<211> 17
<212> DNA
<213> Artificial Sequence

<220>
<223> Description of Artificial Sequence: Synthetic
      primer

<400> 140
tgccaccaat ctctttc                                                    17


<210> 141
<211> 17
<212> DNA
<213> Artificial Sequence

<220>
<223> Description of Artificial Sequence: Synthetic
      primer

<400> 141
aaaatgccac caatctc                                                    17


<210> 142
<211> 17
<212> DNA
<213> Artificial Sequence

<220>
<223> Description of Artificial Sequence: Synthetic
      primer

<400> 142
gaaaatgcca ccaatct                                                    17


<210> 143
<211> 17
<212> DNA
<213> Artificial Sequence

<220>
<223> Description of Artificial Sequence: Synthetic
      primer

<400> 143
agaaaatgcc accaatc                                                    17


<210> 144
<211> 18
<212> DNA
<213> Artificial Sequence

<220>
<223> Description of Artificial Sequence: Synthetic
      primer

<400> 144
tatgaagctg aaaatgga                                                   18

<210> 145
<211> 18
<212> DNA
<213> Artificial Sequence

<220>
<223> Description of Artificial Sequence: Synthetic
      primer

<400> 145
gctatgaagc tgaaaatg                                                    18


<210> 146
<211> 17
<212> DNA
<213> Artificial Sequence

<220>
<223> Description of Artificial Sequence: Synthetic
      primer

<400> 146
cgctatgaag ctgaaaa                                                     17


<210> 147
<211> 16
<212> DNA
<213> Artificial Sequence

<220>
<223> Description of Artificial Sequence: Synthetic
      primer

<400> 147
ccgctatgaa gctgaa                                                      16


<210> 148
<211> 17
<212> DNA
<213> Artificial Sequence

<220>
<223> Description of Artificial Sequence: Synthetic
      primer

<400> 148
acacgaggag taaacta                                                     17


<210> 149
<211> 18
<212> DNA
<213> Artificial Sequence

<220>
<223> Description of Artificial Sequence: Synthetic
      primer

<400> 149
aacacgagga gtaaacta                                                    18


<210> 150
<211> 16
<212> DNA
<213> Artificial Sequence

<220>
<223> Description of Artificial Sequence: Synthetic
      primer

<400> 150
gcaacacgag gagtaa                                                      16


<210> 151
<211> 16
<212> DNA
<213> Artificial Sequence

<220>
<223> Description of Artificial Sequence: Synthetic
      primer

<400> 151
tgcaacacga ggagta                                                      16


<210> 152
<211> 17
<212> DNA
<213> Artificial Sequence

<220>
<223> Description of Artificial Sequence: Synthetic
      primer

<400> 152
attcaactac gccaaga                                                     17


<210> 153
<211> 15
<212> DNA
<213> Artificial Sequence

<220>
<223> Description of Artificial Sequence: Synthetic
      primer

<400> 153
caccggtgga aagca                                                       15


<210> 154
<211> 16
<212> DNA
<213> Artificial Sequence

<220>
<223> Description of Artificial Sequence: Synthetic

primer

<400> 154
ttccaaccgc tgataa                                                        16


<210> 155
<211> 16
<212> DNA
<213> Artificial Sequence

<220>
<223> Description of Artificial Sequence: Synthetic
      primer

<400> 155
tttccaaccg ctgata                                                        16


<210> 156
<211> 17
<212> DNA
<213> Artificial Sequence

<220>
<223> Description of Artificial Sequence: Synthetic
      primer

<400> 156
gtttccaacc gctgata                                                       17


<210> 157
<211> 16
<212> DNA
<213> Artificial Sequence

<220>
<223> Description of Artificial Sequence: Synthetic
      primer

<400> 157
agtcccgagt ttccaa                                                        16


<210> 158
<211> 16
<212> DNA
<213> Artificial Sequence

<220>
<223> Description of Artificial Sequence: Synthetic
      primer

<400> 158
cagtcccgag tttcca                                                        16


<210> 159
<211> 18
<212> DNA
<213> Artificial Sequence

<220>
<223> Description of Artificial Sequence: Synthetic
      primer

<400> 159
cccataattg ggattcac                                                    18


<210> 160
<211> 18
<212> DNA
<213> Artificial Sequence

<220>
<223> Description of Artificial Sequence: Synthetic
      primer

<400> 160
ctcccataat tgggattc                                                    18


<210> 161
<211> 17
<212> DNA
<213> Artificial Sequence

<220>
<223> Description of Artificial Sequence: Synthetic
      primer

<400> 161
agcctctccc ataattg                                                     17


<210> 162
<211> 16
<212> DNA
<213> Artificial Sequence

<220>
<223> Description of Artificial Sequence: Synthetic
      primer

<400> 162
cgagcctctc ccataa                                                      16


<210> 163
<211> 18
<212> DNA
<213> Artificial Sequence

<220>
<223> Description of Artificial Sequence: Synthetic
      primer

<400> 163
gagattcgat ggtcaaag                                                    18


<210> 164
<211> 17
<212> DNA

<213> Artificial Sequence

<220>
<223> Description of Artificial Sequence: Synthetic
      primer

<400> 164
tgagattcga tggtcaa                                                    17


<210> 165
<211> 17
<212> DNA
<213> Artificial Sequence

<220>
<223> Description of Artificial Sequence: Synthetic
      primer

<400> 165
caacaggtgg tgaaaat                                                    17


<210> 166
<211> 17
<212> DNA
<213> Artificial Sequence

<220>
<223> Description of Artificial Sequence: Synthetic
      primer

<400> 166
ggtcagttca tcgtaca                                                    17


<210> 167
<211> 15
<212> DNA
<213> Artificial Sequence

<220>
<223> Description of Artificial Sequence: Synthetic
      primer

<400> 167
ccagctggtg ctgtt                                                      15


<210> 168
<211> 18
<212> DNA
<213> Artificial Sequence

<220>
<223> Description of Artificial Sequence: Synthetic
      primer

<400> 168
tcattagttt gggaatct                                                   18


<210> 169

<211> 19
<212> DNA
<213> Artificial Sequence

<220>
<223> Description of Artificial Sequence: Synthetic
      primer

<400> 169
tttcattagt ttgggaatc                                                    19


<210> 170
<211> 19
<212> DNA
<213> Artificial Sequence

<220>
<223> Description of Artificial Sequence: Synthetic
      primer

<400> 170
ctttcattag tttgggaat                                                    19


<210> 171
<211> 24
<212> DNA
<213> Artificial Sequence

<220>
<223> Description of Artificial Sequence: Synthetic
      probe

<400> 171
cctggccaaa caacactgaa actg                                              24


<210> 172
<211> 19
<212> DNA
<213> Artificial Sequence

<220>
<223> Description of Artificial Sequence: Synthetic
      primer

<400> 172
tcattagttt gggaatctg                                                    19


<210> 173
<211> 24
<212> DNA
<213> Artificial Sequence

<220>
<223> Description of Artificial Sequence: Synthetic
      probe

<400> 173
aagattcagc agctaccgca gccc                                              24

<210> 174
<211> 150
<212> DNA
<213> Artificial Sequence

<220>
<223> Description of Artificial Sequence: Synthetic
      polynucleotide

<400> 174
cgtcgttcgt gaatcctatg aaaccaaatg gacttatgaa accaaatgga ctgatatctt      60

ggcagcatag cgagtaactc ctcaacctgg agttccacct gaagaagcag gggctgcggt     120

agctgctgaa tcttcgtatc cactgcaggt                                      150


<210> 175
<211> 127
<212> DNA
<213> Artificial Sequence

<220>
<223> Description of Artificial Sequence: Synthetic
      polynucleotide

<400> 175
cgtcgttcgt gaatccacag aggctcgcct ttgaccatcg ttgtacgatg aactgaccaa      60

gcttacgcag gctgtatgca tgcattgaga attccgccgc tgggaatgcg ccgtatccac     120

tgcaggt                                                               127


<210> 176
<211> 23
<212> DNA
<213> Artificial Sequence

<220>
<223> Description of Artificial Sequence: Synthetic
      primer

<400> 176
tcgttcgtga atcccaatta tgg                                              23


<210> 177
<211> 25
<212> DNA
<213> Artificial Sequence

<220>
<223> Description of Artificial Sequence: Synthetic
      probe

<400> 177
aggctcgcct ttgaccatcg aatct                                            25


<210> 178
<211> 20

<212> DNA
<213> Artificial Sequence

<220>
<223> Description of Artificial Sequence: Synthetic
      primer

<400> 178
gcggtcagtt catcgtacac                                                    20


<210> 179
<211> 25
<212> DNA
<213> Artificial Sequence

<220>
<223> Description of Artificial Sequence: Synthetic
      probe

<400> 179
agaggctcgc ctttgaccat cgaat                                              25


<210> 180
<211> 24
<212> DNA
<213> Artificial Sequence

<220>
<223> Description of Artificial Sequence: Synthetic
      primer

<400> 180
gtcgttcgtg aatcccaatt atgg                                               24


<210> 181
<211> 21
<212> DNA
<213> Artificial Sequence

<220>
<223> Description of Artificial Sequence: Synthetic
      primer

<400> 181
ggtcagttca tcgtacaccg a                                                  21


<210> 182
<211> 24
<212> DNA
<213> Artificial Sequence

<220>
<223> Description of Artificial Sequence: Synthetic
      primer

<400> 182
cgtcgttcgt gaatcccaat tatg                                               24

<210> 183
<211> 22
<212> DNA
<213> Artificial Sequence

<220>
<223> Description of Artificial Sequence: Synthetic
      primer

<400> 183
ccaagaaatc gtgacactgg ac                                                22


<210> 184
<211> 25
<212> DNA
<213> Artificial Sequence

<220>
<223> Description of Artificial Sequence: Synthetic
      probe

<400> 184
ctcgttgatg tgagaattcc gccgc                                             25


<210> 185
<211> 20
<212> DNA
<213> Artificial Sequence

<220>
<223> Description of Artificial Sequence: Synthetic
      primer

<400> 185
gcagtggata cgagagccat                                                   20


<210> 186
<211> 18
<212> DNA
<213> Artificial Sequence

<220>
<223> Description of Artificial Sequence: Synthetic
      primer

<400> 186
cagacgcagg ctgtatgc                                                     18


<210> 187
<211> 21
<212> DNA
<213> Artificial Sequence

<220>
<223> Description of Artificial Sequence: Synthetic
      probe

<400> 187
ttcccagcgg cggaattctc a                                                 21

<210> 188
<211> 19
<212> DNA
<213> Artificial Sequence

<220>
<223> Description of Artificial Sequence: Synthetic
      primer

<400> 188
acctgcagtg gatacgaga                                                    19


<210> 189
<211> 22
<212> DNA
<213> Artificial Sequence

<220>
<223> Description of Artificial Sequence: Synthetic
      probe

<400> 189
cccagcggcg gaattctcac at                                                22


<210> 190
<211> 20
<212> DNA
<213> Artificial Sequence

<220>
<223> Description of Artificial Sequence: Synthetic
      primer

<400> 190
acctgcagtg gatacgagag                                                   20


<210> 191
<211> 18
<212> DNA
<213> Artificial Sequence

<220>
<223> Description of Artificial Sequence: Synthetic
      primer

<400> 191
tggacgtggt gtcagacg                                                     18


<210> 192
<211> 17
<212> DNA
<213> Artificial Sequence

<220>
<223> Description of Artificial Sequence: Synthetic
      primer

<400> 192
acgtggtgtc agacgca                                                        17


<210> 193
<211> 15
<212> DNA
<213> Artificial Sequence

<220>
<223> Description of Artificial Sequence: Synthetic
      primer

<400> 193
acgcaggctg tatcg                                                          15


<210> 194
<211> 10
<212> DNA
<213> Artificial Sequence

<220>
<223> Description of Artificial Sequence: Synthetic
      probe

<400> 194
aaacacgtgc                                                                10


<210> 195
<211> 10
<212> DNA
<213> Artificial Sequence

<220>
<223> Description of Artificial Sequence: Synthetic
      probe

<400> 195
ccttgttcca                                                                10


<210> 196
<211> 10
<212> DNA
<213> Artificial Sequence

<220>
<223> Description of Artificial Sequence: Synthetic
      probe

<400> 196
cagggacgat                                                                10

## Description

### BACKGROUND

Human cytomegalovirus (CMV) and Epstein-Barr virus (EBV) are members of the human herpes virus family and are among the most prevalent viruses affecting humans known today. Worldwide infection rates for the adult population are estimated to be between 50-80% for CMV and greater than 95% for EBV. Despite their prevalence, these viruses are rarely associated with severe disease in healthy individuals. With a primary infection, individuals may develop mononucleosis (with EBV) or experience mononucleosis-like symptoms (with CMV), but more often are asymptomatic. Though benign for most, an important exception is the immunocompromised population (*e.g*., neonates, elderly, HIV infected individuals and those who have developed AIDS, and individuals who are immunosuppressed or undergoing immunosuppressive therapy, such as bone marrow transplant and solid organ transplant patients and individuals with Crohn's disease, rheumatoid arthritis, or system lupus erythematosus) where infections are a major concern and can have life-threatening consequences.

CMV and EBV target different tissue types in the host and, thus, have different clinical manifestations from one another. CMV has a broad tissue tropism and is usually associated with retinitis, pneumonitis, encephalitis, hepatitis, and gastrointestinal disease. In addition, with neonates, CMV infection can cause blindness, hearing loss, and other neurological disorders. EBV, in contrast, is typically associated with lymphoproliferative disorders and neoplasia, though it has also been implicated in cases of myocarditis, encephalitis, pneumonia, mesenteric adenitis, hepatitis, EBV mononucleosis, nasopharyngeal carcinoma and pancreatitis. Acute EBV infection may often lead to a transient depression in the cell-mediated immune response (anergy), which affects an individual's ability to respond to other viral and intracellular bacterial pathogens.

As with other herpes viruses, even after the initial infection is resolved, CMV and EBV are never fully cleared from the body. Viral persistence is linked to the viruses' ability to enter and maintain a latent state within host cells. The herpes genomes code for a class of immunomodulatory proteins that dowregulate the host's immune response to virally infected cells by impairing antigen presentation. Tins is accomplished through a sophisticated program that coordinates suppression of viral transcription and replication and alters the expression of host MHC I receptors, there by modulating the host immune response. In this manner, the viruses are able to escape detection by the host's immune system.

Viral latency is not permanent, and at times of stress or hormonal fluctuation, the virus can reactivate and begin replicating again (*i.e*., switch to a reproductive program). Viral reactivation from latency results in an increase in circulating virus and re-infection of other host tissues, until the host immune response is activated and the virus can be suppressed again by the host. Viral latency tends to cycle with periods of viral reactivation throughout the host's life.

Virus reactivation is a major concern for the transplant population. Immunosuppressive therapy for prevention of graft rejection predisposes the individual to infection. Before the advent of rapid molecular testing, CMV and EBV disease, from reactivation or primary infection, was, a major cause of graft rejection, morbidity and mortality. CMV infection has also been shown to increase the risk of opportunistic infections and mortality.

With the immunocompromised population, immune responses are depressed and reactivation of a latent virus can result in an infection, causing serious problems. Regular viral load quantitation has now become a critical tool in the management of transplantation patients. This ability to quantitate circulating viral levels allows the clinician to periodically monitor the patient for significant changes in amounts, of circulating virus. This practice allows the clinician to intervene in the early stages of an infection, significantly reducing disease severity and decreasing the potential for damage to the grafted organ.

CMV and EBV testing relied on the culture of the individual viruses. Culturing is a slow process, often taking days to obtain a result, and is known for poor sensitivity (false negatives). The adoption of polymerase chain reaction (PCR)-based tests has led to tests with significantly better sensitivity and turn-around time. Rapid molecular tests have dramatically improved the diagnosis of CMV and EBV infections and are now considered the standard of care for viral detection, especially in situations when speed is critical, *e.g*., diagnosis of central nervous system (CNS) infections.

Quantitative PCR assays have allowed for accurate viral load measurements. The possible correlation between viral load and the risk of disease has advanced the need to track a patient's circulating levels of CMV and EBV by serial testing. This allows the clinician to observe changes in levels of virus over time, permitting the clinician to begin pre-emptive therapy, even before the symptoms of disease are apparent. Viral load measurements for CMV are used to monitor for CMV reactivation, as well as to monitor the patient's response to antiviral treatment. Early diagnosis leads to early treatment, reducing the effects of CMV disease, such as congenital, prenatal, and perinatal CMV infection, CMV mononucleosis, post-transfusion CMV, CMV pneumonitis, and CMV GI disease and CMV retinitis. Treatment for CMV-related diseases includes Cytomegalovirus Immune Globulin Intravenous (Human) (CMV-IGIV). CMV-IGIV is an IgG containing an antibody to Cytomegalovirus (CMV). CMV-IGIV administered alone, or in combination within an antiviral, may be used for CMV-related disease associated with kidney, lung, liver, pancreas and heart transplantation. Ganciclovir and Valganciclovir are antiviral drugs used for treatment of CMV-related diseases. EBV viral load measurements assist in the identification of patients that have a greater risk for developing post-transplant lymphoproliferative disease (PTLD). Treatment for EBV-related diseases includes monitoring antilymphocyte monoclonal antibodies or Valganciclovir.

A rapid and accurate diagnostic test for the detection, screening, quantitation, isolation and sequencing of CMV and EBV, therefore, would provide clinicians with and effective tool for identifying patients at risk for developing CMV and EBV-associated diseases and subsequently supporting effective treatment regimens.

### SUMMARY

The present invention relates to nucleic acid probes and primers for detecting, screening, quantitating, isolating and sequencing viral genetic material from cytomegalovirus and Epstein-Barr virus, and methods for using the probes and primers.

In one embodiment, the present invention is directed to an isolated polynucleotide, comprising a nucleotide sequence selected from the group consisting of: SEQ ID NOS: 1-191.

In another embodiment the present invention is directed to a singleplex primer set or collection of primer sets for amplifying CMV DNA, comprising a nucleotide sequence selected from the group consisting of: (1) SEQ ID NOS: 33 and 166; (2) SEQ ID NOS: 27 and 155;(3) SEQ ID NOS: 27 and 156; (4) SEQ ID NOS: 33 and 164; (5) SEQ ID NOS: 36 and 167; (6)SEQ ID NOS: 20 and 144; (7) SEQ ID NOS: 20 and 145; (8) SEQ ID NOS: 30 and 159; (9) SEQ ID NOS: 35 and 167; (10) SEQ ID NOS: 30 and 160; (11) SEQ ID NOS: 20 and 146; (12) SEQ ID NOS: 20 and 147; (13) SEQ ID NOS:21 and 146; (14) SEQ ID NOS: 27 and 154; (15) SEQ ID NOS: 21 and 147; (16) SEQ ID NOS:36 and 172; (17) SEQ ID NOS: 36 and 170; (18) SEQ ID NOS:21 and 145; (19) SEQ ID NOS:36 and 169; (20) SEQ ID NOS: 31 and 163; (21) SEQ ID NOS: 23 and 149; (22) SEQ ID NOS: 27 and 157; (23) SEQ ID NOS: 23 and 148; (24) SEQ ID NOS: 36 and 168; (25) SEQ ID NOS: 27 and 158; (26) SEQ ID NOS: 20 and 148; (27) SEQ ID NOS: 28 and 162; (28) SEQ ID NOS: 29 and 161; (29) SEQ ID NOS: 23 and 150; (30) SEQ ID NOS: 21 and 149; (31) SEQ ID NOS:35 and 170; (32) SEQ ID NOS: 21 and 151; (33) SEQ ID NOS: 176 and 178; (34) SEQ ID NOS: 180 and 181; and (35) SEQ ID NOS: 182 and 181. In another embodiment, the present invention is drawn to a singleplex polynucleotide, probe for binding to CMV DNA, comprising a nucleotide sequence selected from the group consisting of SEQ ID NOS:74-84, 91-100, 103-108, 177 and 179.

In another embodiment, the present invention is directed to a singleplex primer set or collection of primer sets for amplifying EBV DNA, comprising a nucleotide sequence selected from the group consisting of (1) SEQ ID NOS: 14 and 134; (2) SEQ ID NOS:8 and 120; (3) SEQ ID NOS: 6 and 117; (4) SEQ ID NOS: 8 and 122; (5) SEQ ID NOS: 8 and 120; (6) SEQ ID NOS: 19 and 137; (7) SEQ ID NOS: 4 and 115; (8) SEQ ID NOS:7 and 120; (9) SEQ ID NOS:32 and 165; (10) SEQ ID NOS:34 and 165; (11) SEQ ID NOS:6 and 118; (12) SEQ ID NOS:24 and 152; (13) SEQ ID NOS: 11 and 128; (14) SEQ ID NOS:5 and 118; (15) SEQ ID NOS: 9 and 123; (16) SEQ ID NOS: 19 and 139; (17) SEQ ID NOS 6 and 119: (18) SEQ ID NOS: 25 and 152; (19) SEQ ID NOS:19 and 138; (20) SEQ ID NOS:9 and 124; (21) SEQ ID NOS: 5 and 119; (22) SEQ ID NOS:22 and 140; (23) SEQ ID NOS: 15 and 133; (24) SEQ ID NOS:22 and 143; (25) SEQ ID NOS: 7 and 121; (26) SEQ ID NOS: 22 and 142; (27) SEQ ID NOS: 10 and 129; (28) SEQ ID NOS: 10 and 128; (29) 15 and 132; (30) SEQ ID NOS: 3 and 111; (31) SEQ ID NOS: 9 and 122; (32) SEQ ID NOS: 3 and 113; (33) SEQ ID NOS: and 112; (34) SEQ ID NOS: 2 and 110; (35) SEQ ID NOS: 26 and 153; (36) SEQ ID NOS: 18 and 136; (37) SEQ ID NOS: 16 and 135; (38) 9 and 127; (39) SEQ ID NOS: I and 109; (40) SEQ ID NOS: 12 and 130; (41) SEQ ID NOS: 4 and 116; (42) SEQ ID NOS: 8 and 125; (43) SEQ ID NOS: 9 and 126; (44) SEQ ID NOS: 3 and 114; (45) SEQ ID NOS:7 and 125; (46) SEQ ID NOS: 22 and 141; (47) SEQ ID NOS: 13 and 131; (48) SEQ ID NOS: 17 and 136; (49) SEQ ID NOS: 183 and 185; (50) SEQ ID NOS: 186 and 188; (51) SEQ ID NOS:186 and 190; and (52) SEQ ID NOS: 191 and 190. In another embodiment, the present invention is drawn to a singleplex polynucleotide probe for binding to EBV DNA, comprising a nucleotide sequence selected from the group consisting of: SEQ ID NOS: 37-73, 85-90; 101, 102, 171, 184, 187 and 189.

In another embodiment, the present invention is direted to a multiplex set of primer sets for amplifying CMV and EBV DNA simultaneously, comprising, a nucleotide sequence selected from the primer sets consisting of: Groups 1-152 of Table 2. In another embodiment, the present invention is directed to multiplex polynucleotide probes for binding to CMV and EBV DNA, comprising a nucleotide sequence selected from the group consisting of: SEQ ID NOS: 74-84, 91-100, 103-108, 177, 179 (CMV probes) and 37-73, 85-90, 101, 102, 171, 184, 187 and 189 (EBV probes).

In a preferred embodiment, the present invention is drawn to a multiplex set of primer sets for amplifying CMV and EBV DNA simultaneously, comprising (1) SEQ ID NOS: 33 or 176 (forward primers) and 166 or 178 (reverse primers) for amplifying CMV DNA and (2) SEQ ID NOS: 14 or 183 (forward primers) and 34 or 185 (reverse primers) for amplifying EBV DNA. In a preferred embodiment, the present invention is directed to multiplex polynucleotide probes for binding to CMV and EBV DNA, comprising the nucleotide sequences of SEQ ID NOS: 99 or 177 (CMV probes) and 64 or 185 (EBV probes).

In a particular embodiment, the probe(s) is labeled, *e.g*., wherein the probe(s) comprises a detectable moiety selected from the group consisting of: a fluorescent label, a chemiluminescent label, a radioactive label, biotin or gold. The probe(s) also comprises a quencher moiety. In one embodiment, the set of probes for binding to CMV and EBV DNA are each labeled with a different detectable label. In another embodiment, the set of probes for binding to CMV and EBV DNA are labeled with the same detectable label.

The present invention is also directed to a polynuelcotide internal control probe, an internal control plasmid and a CMV/EBV positive control plasmid. The competitive internal control plasmid is a synthetic target that does not occur naturally in clinical sample types for which this assay is intended. The synthetic target sequence incoporates a sequence from the *Arabidopsis thaliana* chloroplast plastid-1 gene. The synthetic target sequence is: 5'-CGTCGTTCGTGAATCCTATGAAACCAAATGGACTTATGAAACCAAATGGACT GATATCTTGGCAGCATAGCGAGTAACTCCTCAACCTGGAGTTCCACCTGAAGAA GCAGGGGCTGCGGTAGCTGCTGAATCTTCGTATCCACTGCAGGT-3' (SEQ ID NO: 174). This internal control plasmid employs a single primer from each of the CMV and EBV primer sets (SEQ ID NOS: 33 and 134, respectively). The internal control probe (SEQ ID NO: 173) is a unique probe that reognizes the synthetic target sequence (SEQ ID NO: 174). A plasmid vector containing the internal control target sequence and its specific probe (SEQ ID NO: 173) are included in the assay. The internal control plasmid may either be added directly to the réaction mix to monitor PCR efficiency or to the sample for use as a process control, to monitor efficiency of both sample extraction and PCR. A non-competitive control may also be used in this invention.

The CMV/EBV positive-control plasmid contains, a partial amplicon sequence for both CMV and EBV. The positive control plasmid comprises the forward primer, probe and reverse primer sequences for CMV, followed by a HindIII restriction site, and the forward primer, probe and reverse primer sequences for EBV. The CMV/EBV positive control plasmid may be used to generate a standard curve to quantitate the amount of CMV and EBV DNA present in a reaction or may be used to confirm that the assay is performing to specifications.

In one embodiment, the present invention is directed to a method of hybridizing one or more nucleic acid sequences comprising SEQ ID NOS: 1-172 and 176-191 to a CMV and/or EBV template or CMV and/or EBV genomic sequence or a CMV and/or EBV sequence derived from an artificial construct, comprising contating the one or more nucleic acid sequences to a sample under conditions suitable for hybridization. In another embodiment, the method further comprises isolating the hybridized CMV and/or EBV sequence. In one embodiment, the method further comprises sequencing the hybridized CMV and/or EBV sequence.

In one embodiment, the present invention is directed to a method of producing a nucleic acid product, comprising contacting one or more nucleic acid sequences ofSEQ ID NOS: 1-172 and 176-191 with a CMV and/or EBV template or CMV and/or EBV genomic sequence or a CMV and/or EBV sequence derived from an artificial construct under conditions suitable for nucleic acid polymerization, In a further embodiment, the nucleic acid product is an amplified product produced using at least one forward primer selected from the group consisting of SEQ ID NOS: 33 and 14 and at least one reverse primer selected from the group consisting of SEQ ID NOS: 166 and 134.

In another embodiment, the present invention is directed to a method for detecting and/or screening and/or quantitating CMV in a sample, comprising (1) contacting at least one forward and reverse primer set selected from the group consisting of: Groups 72-147 of Table 2 to a sample; (2) conducting an amplification process; and (3) detecting the generation of an amplified product, wherein the generation of an amplified product indicates the presence of CMV in the sample. In a particular embodiment, step (3) is performed using a labeled probe comprising a nucleotide sequence selected from the group consisting of SEQ ID NOS: 74-84, 91-100, 103-108, 117 and 179 that hybridizes to one of the strands of the amplicon generated by at least one forward and reserve primer set. The present invention is also directed to a polynucleotide internal control probe (SEQ ID NO: 173), an internal control plasmid and a CMV/EBV positive control plasmid, each of which is listed in Table **3**. A non-competitive control may also be used.

In embodiment, the present invention is directed to a method, for detecting and/or screening and/or quantitating EBV in a sample, comprising (1) at least one forward and reverse primer set selected from the group consisting of: Groups 1-71 and 148-152 of Table 2 to a sample; (2) conducting an amplification process; and (3) detecting the generation of an amplified product, wherein the generation of an amplified product indicates the presence of EBV in the sample. In a particular embodiment, step (3) is performed using a labeled probe comprising a nucleotide sequence selected from the group consisting of: SEQ ID NOS: 37-73, 85-90, 101, 102, 171, 184, 187 and 180 that hybridizes to one of the strands of the amplicon generated by at least one forward and reverse primer set. The present invention is also directed a polynucleotide internal control probe (SEQ ID NO: 173), an internal control plasmid and a CMV/EBV positive control plasmid. A non-competitive control may also be used.

In another embodiment, the present invention is directed to a method for detecting and/or screening and/or quantitating CMV and EBV in a sample simultaneously, comprising (1) contacting at least one multiplex set of a forward and reverse primer set listed in Table 2 to a sample; (2) conducting an amplification process; and (3) detecting the generation of an amplified product, wherein the generation of an amplified product indicates the presence of CMV and BV in the sample. Step (3) is performed using a combination of differently labeled multiplex CMV and EBV probes, or multiplex CMV and EBV probes labeled with the same detectable label. In one embodiment, the primer set and combination of probes is selected from the group consisting of Groups 1-152 of Table 2. In a preferred embodiment, the present invention is drawn to a multiplex set of primer sets for amplifying CMV and EBV DNA simultaneously, comprising (1) SEQ ID NOS: 33 or 176 (forward primers) and 166 or 178 (reverse primers) for amplifying CMV DNA and (2) SEQ ID NOS: 14 or 183 (forward primers) and 134 or 185 (reverse primers) for amplifying EBV DNA, Step (3) is performed using a combination of differently labeled multiplex CMV and EBV probes comprising the nucleotide sequences of SEQ ID NOS: 99 or 177 (CMV probes) and 64 or 185 (EBV probes).

The present invention is also directed to a polynuelcotide internal control probe (SEQ ID NO: 173), an internal control plasmid and a CMV/EBV positive control plasmid of Table 3. A non-competitive control may also be used.

In a particular embodiment the probe(s) is fluorescently labeled and the step of detecting the binding of the probe to the amplified product comprises measuring the fluorescence of the sample. In another embodiment, the probe comprises a fluorescent reporter moiety and a quencher of fluorescence moiety. Upon probe hybridization with the amplified product, the exonuclease activity of a DNA polymerase cleaves the probe reporter and quencher, resulting in the unquenched emission of fluorescence, which is detected. An increase in the amplified product causes a proportional increase in fluoresconce, due to cleavage of the probe and release of the reporter moiety, of the probe. The amplified product is quantified in real time as it accumulates. In another embodiment, each of the probes in the multiplex reaction is labeled with a different distinguishable and detectable label.

In an additional embodiment, the probes are molecular beacons. Molecular beacons are single-stranded probes that form a stem-and-loop structure. A fluorophore is covalently linked to one end of the stem and a quencher is covalently linked to the other end of the stem forming a stem hybrid. When a molecular beacon hybridizes to a target nucleic acid sequence, the probe undergoes a conformational change that results in the dissociation of the stem hybrid and, thus the fluorophore and the quencher move away from each other, enabling the probe to fluoresce brightly, Molecular beacons can be labeled with differently colored fluorophores to detect different target sequences. Any of the probes described herein may be designed and utilized as molecular beacons.

In a particular embodiment, the sample is blood, serum, plasma, enriched peripheral blood mononuclear cells, bone marrow, urine, neoplastic or other tissue obtained from biopsies, cerebrospinal fluid, saliva, fluids collected from the aer, eye, mouth, and respiratory airways, sputum, urine, stool, skin, semen, seminal fluid, gastric secretions, tears, oropharyngeal swabs, nasopharyngeal swabs, throat swabs, nasal aspirates, nasal wash, renal tissue and fluid therefrom including perfusion media, tissues either to be utilized for transplantation between individuals and/or animal derived tissues to be utilized for transplantation into a human recipient fluids and cells obtained by the perfusion of tissues of both human and animal origin, and fluids and cells derived from the culturing of human cells, including human stem cells and human cartilage or fibroblasts. In one embodiment, the sample is from a haman. In the sample is non-human. In a further embodiment, the sample is derived from an inanimate object.

In one embodiment, the present invention is directed to a kit for detecting and/or screening and/or quantitating CMV and/or EBV DNA, comprising: a) at least one forward primer comprising the sequence selected from the group consisting of; SEQ ID NOS: 1-36, 176, 180, 182, 183, 186 and 191; b) at least one reverse primer comprising the sequence selected from the group consisting of: SEQ ID NOS: 109-170, 172, 178, 181, 185, 188 and 190; and c) one or more probes comprising a sequence selected from the group consisting of: SEQ ID NOS: 37-108, 171, 177, 179, 184, 187 and 189. In a further embodiment, the one or more probes are labeled with different distinguishable and detectable labels. In another embodiment, the one or more probes are labeled with the same detectable labels. In an additional embodiment, the at least one forward primer, the at least one reverse primer and the one or more probes are selected from the group consisting of: Groups 1-152 of Table 2. The performance of the assay is monitored through the use of a process/internal control that monitors the efficiency of sample extraction and PCR. In addition, a positive control is provided for generation of a standard curve and demonstration of the performance of the assay. The internal control and CMV/EBV positive control are specified in Table 3. A non-competitive control may also be used.

In a further embodiment the present invention is directed to a kit for amplifying and sequencing CMV and/or EBV DNA, comprising: a) at least one forward primer comprising the sequence selected from the group consisting of: SEQ ID NOS: 1-36, 176, 180, 182, 183, 186 and 191; b) at least one reverse primer comprising the sequence selected from the group consisting of: SEQ ID NOS: 109-170, 172, 178, 181, 185, 188 and 190; c) at least one forward sequencing primer comprising the sequence selected from the group consisting of: SEQ ID NOS: 1-36, 176, 190, 182, 183, 186 and 191 for use as a sequencing primer; d) at least, one reverse sequencing primer comprising the sequence selected from the group consisting of: SEQ ID NOS: 109-170, 172, 178, 181, 185, 188 and 190 for use as a sequencing primer; and reagents for the sequencing of amplified DNA fragments using either standard sequencing chemistries, for example, dye labeled terminator or dye labeled primer.

In one embodiment, the present invention is directed to a method for diagnosing a CMV and/or EBV-associated condition, syndrome of disease, comprising: a) contacting a sample with at least one forward and reverse primer set selected from the group consisting of: Groups 1-152 of Table 2; b) conducting an amplification process; and c) detecting and quantitating the generation of an amplified product using one or more probes selected from the group consisiting of: SEQ IN NOS: 37-108, 171, 177, 179, 184, 187 and 189; wherein the generation of an amplified product indicates the presence of CMV and/or EBV in the sample. The sample is selected from the group consisting of: blood, serum, plasma, enriched peripheral blood mononuclear cells, bone marrow, urine, neoplastic or other tissue obtained from biopsies, cerebrospinal fluid, saliva, fluids collected from the car, eye, mouth, and respiratory airways, sputum, urine, stool, skin, semen, seminal fluid, gastric secretions, tears, oropharyngeal swabs, nasopharyngeal swabs, throat swabs, nasal aspirates, nasal wash, renal tissue and fluid therefrom inluding perfusion media, tissues either to be utilized for transplantation between individuals and/or animal derived tissues to be utilized for transplantation into a human recipient, fluids and cells obtained by the perfusion of tissues of both human and animal origin, and fluids and cells derived from the culturing of human cells, including human stem cells and human cartilage or fibroblasts. The CMV-associated condition, syndrome or disease is selected from the group consisting of, but not limited to: congenital and perinatal CMV infection, mononucleosis, post-transfusion CMV, solid organ transplantation, hematopoietic stem cell transplantation, retinitis, pneumonitis, encephalitis, hepatitis, gastrointestinal disease, blindness, hearing loss, and other neurogical disorders. The EBV-assosiated condition, syndrome or disease is selected from the group consisting of, but not limited to: lymphoproliferative disorders, neoplasia, myocarditis, encephalitis, pneumonia, mesenteric adenitis, hepatitis, EBV mononucleosis, nasopharyngeal carcinoma and pancreatitis.

In another embodiment, the present invention is directed to a method for diagnosing a CMV and/or EBV-associated condition, syndrome or disease, comprising: a) contacting a denatured target sequence from a sample with at least one probe selected from the group consisting of: SEQ ID NOS: 37-108, 171, 117, 179, 184, 187 and 189 for hybridization to occur; wherein the hybridization indicates the presence of CMV and/or EBV in the sample.

The oligonucleotides of the present invention and their resulting amplicons do not cross react and, thus, will work together without negatively impacting either of the individual/singleplex assays. The primers and probes of the present invention also do not cross react with other herpes virus family members.

### BRIEF DESCRIPTION OF THE DRAWINGS

For a better understanding of the invention and to show how the same may be carried into effect reference is now made by way of example to the accompanying drawings in which:

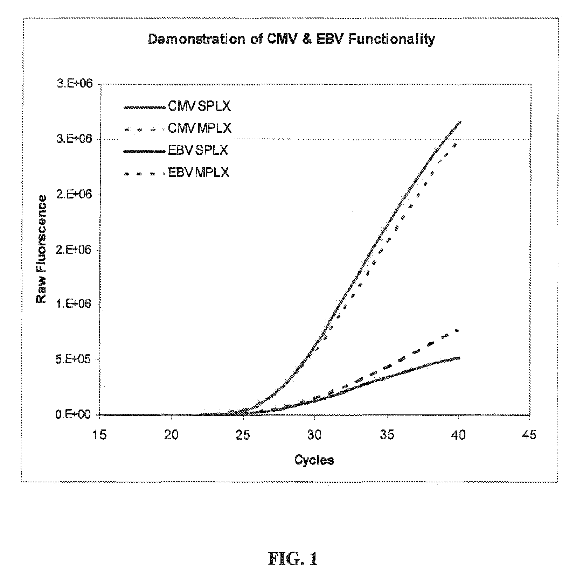

FIG. 1 shows the functionality of the CMV and EBV primer-probe sets, wherein primer-probe sets for CMV and EBV were used on genomic DNA from respective organisms in singleplex (CMV or EBV) and multiplex format (CMV and EBV).

### DETAILED DESCRIPTION

The primers and probes of the present invention can be used in singleplex format for the detection and/or screening and/or quantitation of CMV or EBV, or combined in a multiplex format to allow for the detection and/or screening and/or quantitation of CMV and EBV DNA simultaneously, without loss of assay precision or sensitivity. Currently, EBV post-transplant lymphoproliferative disease (PTLD) and CMV reactivation/disease are tested separately, however, the multiplex format option of the present invention allows relative comparisons to be made between these two prevalent pathogens. The primers and probes of the present invention can be used for viral burden monitoring of solid organ or hematopoietic stem cell transplant patients, the evaluation of antiviral therapy efficacy, the presence of circulating CMV and/or EBV, the presence of CMV and/or EBV proviral DNA and/or as a diagnostic reagent for CMV and EBV-associated diseases, syndromes and conditions.

Commercial singleplex PCR tests for CMV and EBV have been used in clinical, laboratories, however, a method for simultaneously quantifying both these viruses is not presently available. The advantages of such a multiplex format are: (1) simplified and improved testing and analysis; (2) increased efficiency and cost-effectiveness; (3) the relationship of the CMV and EBV viruses can provide more information regarding the clinical state of the patient, and allow for inter-organism comparisons to be made; (4) decreased turnaround time (increased speed of reporting results); (5) increased productivity (less equipment time needed); (6) coordination/standardization of results for patients for multiple organisms (reduces error from inter-assay variation); and (7) prognostic value - the viral burden of each virus can be assessed, which gives a better indication of the health of the patient and the state of his/her immune system to tailor treatment regimen (antiviral versus change in dosage of immunosuppressant).

Current nucleic acid diagnostic testing kits are cannot adequately identify the broad genetic diversity of target CMV and EBV pathogens. A rapid and accurate diagnostic test for the detection and quantitation of CMV and EBV, therefore, regardless of the specific infecting virus, would support effective treatments and control of infection. The treatments for CMV and EBV infection will depend upon the clinical disease state of the patient. Most likely, the treatment will be different for patients having a disease, syndrome or condition associated with CMV and/or EBV.

Described herein are nucleic acid primers and probes for detecting and/or screening viral genetic material, especially CMV and ERV, and methods for designing and optimizing the respective primer and probe sequences. The present invention therefore provides a method for specifically detecting and/or screening for the presence of CMV and/or EBV in a given sample using the primers and probes provided herein. Of particular interest in this regard is the ability of the disclosed primers and probes - as well as those that can be designed according to the disclosed methods - to specifically detect or screen all or a majority of presently characterized strains of CMV and EBV. The optimized primers and probes of the invention are useful, therefore, for identifying and diagnosing the causative or contributing agents of disease caused by CMV and EBV, whereupon an appropriate treatment can then be administered to the individual to eradicate the viruses.

The present invention provides one or more sets of primers that can anneal to all currently identified strains of CMV and EBV and thereby amplify a target from a biological sample. The present invention provides at least first primer and at least a second primer for CMV and EBV, each of which comprises a nucleotide sequence designed according to the invention principles disclosed herein, which are used together to positively identify the presence of CMV or EBV in a sample in a singleplex assay, or CMV and EBV in a sample in a multiplex assay, regardless of the actual nucleotide composition of the infecting CMV and/or EBV strain(s). Of note, each of the primer sequences can used as a probe to detect viral strains.

Also provided by the present invention are probes that hybridize to CMV and/or EBV sequences and/or amplified products derived from CMV and/or EBV sequences. A probe can be labeled, for example, such that when it binds to an amplified or unamplified target sequence, or after it has been cleaved after binding, a fluorescent signal is emitted that is detectable under various spectroscopy and light-measurement apparatus. The use of a labeled probe, therefore, can enhance the sensitivity of detection of a target in an amplification reaction of CMV and EBV DNA because it permits the detection and quantitation of viral derived DNA at low template concentrations that might not be conducive to visual detection as a gel-stained amplification product.

The present invention also provides multiplex panels that can be utilized for screening at least three important disease categories. One of the panels includes screening individuals for CMV in combination with any of the following organisms for "TORCH" infections: *Toxoplasma gondii*; Other, including hepatitis B virus, HIV, syphilis, varicella zoster virus, parvovirus B19; Rubella; CMV; and Herpes simplex virus.

An additional multiplex panel is provided that involves a perinatal screen. This multiplex assay can be utilised to screen for CMV in combination with any of the lowing organisms: *Listeria* monocytogenes, *E*. *coli,* Group B *Streptococcus,* Herpes simplex virus-1, Herpes simplex virus-2, *Neisseria gonorrhoeae* and *Chlamydia trachomatis.*

A further multiplex panel is provided that involves a screen for organisms associated with the symptoms of fever, sore throat and fatigue. Specifically, this panel can be utilized to screen individuals for EBV in combination with any of the following organisms: Group A *Streptococcus,* CMV, *Toxoplasma gondii,* measles, leukemia, common cold (rhinovirus, coronavirus, parainfluenza), influenza, adenovirus, *Corynebacterium diphtheriae, Neisseria gonorrhoeae, C*. *pneumoniae, Mycoplasma pneumoniae, Candida albicans* and *Fusobacterium necrophorum.*

The present invention also provides multiplex assays that can be utilized for the detection of mutations conferring drug resistance in CMV and/or EBV, such as mutations in the UL54 and UL97 genes. Other mutations in CMV and or EBV genes may be detected using the multiplex assays. Many different molecular methods of detection are available, including those methods known to one of skill in the art. Such methods include microarrays, sequencing, beads, mass spectrometry, flow cytometry, hybridization to a filter or other matrix, or other methods that identify targets based on molecular weight or nucleotide sequence.

Primers and probes of the invention are sequences that anneal to a viral genomic or viral genomic derived sequence, *e.g*., CMV and EBV (the "target"). The target sequence can be, for example, a viral genome or a subset "region", of a viral genome. In one embodiment, the entire genomic sequence can be "scanned" for optimized primers and probes useful for detecting viral strains. In other embodiments, particular regions of the viral genome can be scanned, *e.g.,* regions that are documented in the literature as being useful for detecting multiple strains, regions that are conserved, or regions where sufficient information is available in, for example, a public database, with respect to viral strains.

Sets or groups of primers and probes are generated based on the target to be detected. The set of all possible primers and probes can include, for example, sequences that include the variability at every site based on the known viral strains, or the primers and probes can be generated based on a consensus sequence of the target. The primers and probes are generated such that the primers and probes are able to anneal to a particular strain or a consensus sequence under high stringency conditions. For example, one of skill in the art recognizes that for any particular sequence, it is possible to provide more than one oligonucleotide sequence that will anneal to the particular target sequence, even under high stringency conditions. The set of primers and probes to be sampled for the purposes of the present invention includes, for example, all such oligonuelcotides for all viral strain sequences. Alternatively, the primers and probes include all such oligonucleotide for a given consensus sequence for a target.

Typically, stringent hybridization and washing conditions are used for nucleic acid molecules over about 500 bp. Stringent hybridization conditions include a solution comprising about 1 M Na⁺ at 25°C to 30°C below the Tm; *e.g*., 5 × SSPE, 0.5% SDS, at 65°C; *see*, Ausubel, et al., Current Protocols in Molecular Biology, Greene Publishing, 1995; Sambrook et al., Molecular Cloning: A Laboratory Manual, Cold Spring Harbor Press, 1989). Tm is dependent on both the G+C content and the concentration of salt ions, *e.g*., Na⁺ and K⁺. A formula to calculate the Tm of nucleic acid molecules greater than aBout 500 bp is Tm = 81.5 + 0,41 (%(G+G)) - log₁₀[Na⁺]. Washing conditions are generally performed at least at equivalent stringency conditions as the hybridization. If the background levels are high, washing can be performed at higher stringency, such as around 15°C below the Tm.

The set of primers and probes, once determined as described above, are optimized for hybridizing to a plurality of viral strains by employing scoring and/or ranking steps that provide a positive or negative preference or "weight" to certain nucleotides in a target nucleic acid strain sequence. For example, if a consensus sequence is used to generate the full set of primers and probes, then a particular primers sequence is scored for its ability to anneal to the corresponding sequence of every known native strain sequence. Even if a probe were originally generated based on a consensus, therefore, the validation of the probe is in its ability to specifically anneal and detect every, or a large majority of, viral strain sequences. The particular scoring or ranking, steps performed depend upon the intended use for the primer and/or probe, the particular target nucleic acid sequence, and the number of strains of that target nucleic acid sequence. The methods of the invention provide optimal primer and probe sequences because they hybridize to all or a subset of CMV and EBV strains. Once optimized oligonucleotides are identified that can anneal to viral strains, the sequences can then further be optimized for use, for example, in conjunction with another optimized sequence as a "primer set" or for use as a probe. A "primer set" is defined as at least one forward primer and one reverse primer.

Primer or probe sequences can be ranked according to specific hybridization parameters or metrics that assign a score value indicating their ability to anneal to viral strains under highly stringent conditions. Where a primer set is being scored, a "first" or "forward" a primer is scored and the "second" or "reverse"-oriented primer sequences can be optimized similarly but with potentially additional parameters, followed by an optional evaluation for primer dimmers, for example, between the forward and reverse primers.

The scoring or ranking steps that are used in the methods of determining the primers and probes includes, for example, the following parameters: a target sequence score for the target nucleic acid sequence(s), *e.g*., the PriMD^{®} score; a mean conservation score for the target nucleic acid sequence(s); a mean coverage score for the target nucleic acid scquence(s); 100% conservation score of a portion (*e.g*., 5' end, center, 3' end) of the target nucleic acid sequence(s); a species score; a strain score; a subtype score; a serotype score; an associated disease score; a year score; a country of origin score; a duplicate score; a patent score; and a minimum qualifying score. Other parameters that are used iuclude, for example, the number of mismatches, the number of critical mismatches (*e.g*., mismatches that result in the predicted failure of the sequence to anneal to a target sequence), the number of native strain sequences that contain critical mismatches, and predicted Tm values. The term "Tm" refers to the temperature at which a population of double-stranded nucleic acid molecules becomes half-dissociated into single strands. Methods for calculating the Tm of nucleic acids are well known in the art (Berger and Kimmel (1987) Meth. Enzymol., Vol. 152: Guide To Molecular Cloning Techniques, San Diego: Academic Press, Inc. and Sambrook et al. (1989) Molecular Cloning: A Laboratory Manual, (2nd ed.) Vols. 1-3, Cold Spring Harbor Laboratory).

The resultant scores represent steps in determining nucleotide or whole target nucleic acid sequence preference, while tailoring the primer and/or probe sequences so that they hybridize to a plurality of target nucleic acid strains. The methods of determining the primers and probes also can comprise the step of allowing for one or more nucleotide changes when determining identity between the candidate primer and probe sequences and the target nucleic acid strain sequences, or their complements.

In another embodiment, the methods of determining the primers and probes comprise the steps of comparing the candidate primer and probe nucleic acid sequences to "exclusion nucleic acid sequences" and then rejecting those candidate nucleic acid sequences that share identity with the exclusion nucleic acid sequences. In another embodiment, the methods of the invention comprise the steps of comparing the candidate a primer and probe nucleic acid sequence to "inclusion nucleic acid sequences" and then rejecting those candidate nucleic acid sequences that do not share identity with the inclusion nucleic acid sequences.

In other embodiments of the methods of determining the primers and probes, optimizing primers and probes comprises using a polymerase chain reaction (PCR) penalty score formula comprising at least one of a weighted sum of: primer Tm - optimal Tm; difference between primer Tms; amplicon length - minimum amplicon length; and distance between the a primer and a TaqMan^{®} probe. The optimizing step also can comprise determining the ability of the candidate sequence to hybridize with the most target nucleic acid strain sequences (*e.g*., the most target organisms or genes). In Another embodiment, the selecting or optimizing step comprises determining which sequences have mean conservation scores closest to 1, wherein a standard of deviation on the mean conservation scores is also compared.

In other embodiments, the methods further comprise the step of evaluating which target nucleic acid strain sequences are hybridized by an optimal forward primer and an optimal reverse primer, for example, by determining the number of base ditterences between target nucleic acid strain sequences in a database. For example, the evaluating step can comprise performing an *in silico* polymerase chain reaction, involving (1) rejecting the forward a primer and/or reverse primer if it does not meet inclusion or exclusion criteria; (2) rejecting the forward primer and/or reverse primer if it does not amplify a medically valuable nucleic acid; (3) conducting a BLAST analysis to identity forward a primer sequences and/or reverse primer sequences that overlap with a published and/or patented sequence; (4) and/or determining the secondary structure of the forward primer; reverse primer, and/or target. In an embodiment, the evaluating step includes evaluating whether the forward primer sequence, reverse primer sequence, and/or probe sequence hybridizes to sequences in the database other than the nucleic acid sequences that are representative of the target strains.

The present invention provides polynucleotides that have preferred primer and probe qualities. These qualities are specific to the sequences of the optimized probes, however, one of skill in the art would recognize that other molecules with similar sequences could also be used. The oligonucleotides provided herein comprise a sequence that shares at least about 60-70% identity with a sequence described in Table 2. In Addition, the sequences can be incorporated into longer sequences, provided they function to specifically anneal to and identify viral strains. In another embodiment, the invention provides a nucleic acid comprising a sequence that shares at least about 71 %, about 72%, about 73%, about 74%, about 73%, about 76%, about 77%, about 78%, about 79%, about 80%, about 81%, about 82%, about 83%, about 84%, about 85%, about 86%, about 87% about 88%, about 89%, about 90%, about 91%, about 92%, about 93%, about 94%, about 95%, about 96%, about 97%, about 98%, about 99%, or about 100% identity with the sequences of Table 2 or complement thereof. The terms "homology" or "identity" or "similarity" refer to sequence relationships between two nucleic acid molecules and can be determined by comparing a nucleotide position in each sequence when aligned for purposes of comparison. The term "homology" refers to the relatedness of two nucleic acid or protein sequences. The term "identity" refers to the degree to which nucleic acids are the same between two sequences. The term "smilarity" refers to the degree to which nucleic acids are the same, but includes neutral degenerate nucleotides that can be substituted within a codon without changing the amino acid identity of the codon, as is well known in the art. The primer and/or probe nucleic acid sequences of the invention are complementary to the target nucleic acid sequence. The probe and/or primer nucleic acid sequences of the invention are optimal for identifying numerous strains of a target nucleic acid, *e.g*., from CMV and/or EBV pathogens. In an embodiment, the nucleic acids of the invention are primers for the synthesis (*e.g*., amplification) of target nucleic acid strains and/or probes for identification, isolation, detection, quantitation or analysis of target nucleic acid strains, *e.g*., an amplified target nucleic acid strain that is amplified using the primers of the invention.

The present oligonucleotides hybridize with more than one viral strain (strains as determined by differences in their genomic sequence). The probes and primers provided herein can, for example, allow for the detection and quantitation of currently identified viral strains or a subset thereof. In addition, the primers and probes of the present invention, depending on the strain sequence(s), can allow for the detection and quantitation of previously unidentified viral strains. In addition, the primers and probes of the present invention, depending on the strain sequence(s), can allow for the detection and quantitation of previously unknown viral strains. The methods of the invention provide for optimal primers and probes, and sets thereof, and combinations of sets thereof, which can hybridize with a larger number of target strains than available primers and probes.

In other aspects, the invention also provides vectors (*e.g*., plasmid, phage, expression), cell lines (*e.g*., mammalian, insect, yeast, bacteria), and kits comprising any of the sequences of the invention described herein. The invention further provides known or previously unknown target nucleic acid strain sequences that are identified, for example, using the methods of the invention. In an embodiment, the target nucleic acid strain sequence is an amplification product. In another embodiment, the target nucleic acid strain sequence is a native or synthetic nucleic acid. The primers, probes, and target nucleic acid strain sequences, vectors, cell lines, and kits can have any number of uses, such as diagnostic, investigative, confirmatory, monitoring, predictive or prognostic.

A diagnostic kit is provided by the present invention that comprises one or more of the oligonucleotides described herein, which are useful for detecting CMV and/or EBV infection in an individual and/or from a sample. An individual can be a human male, human female, human adult, human child, or human fetus. An individual can also be any mammal, reptile, avian, fish, or amphibian. Hence, an individual can be a mouse, rat, sheep, dog, simian, horse, cattle, chicken, porcine, lamb, bird or fish. A sample includes any item, surface, material, clothing, or environment, for example, sewage or water treatment plants, in which it may be desirable to test for the presence of CMV and/or EBV strains. Thus, for instance, the present invention includes testing door handles, faucets table surfaces, elevator buttons, chairs, toilet seats, sinks, kitchen surfaces, children's cribs, bed linen, pillows, keyboard, and so on, for the presence of CMV and/or EBV strains.

A probe of the present invention can comprise a label such as, for example, a fluorescent label, a chemiluminescent label, a radioactive label, biotin, gold, dendrimers aptamer, enzymes, proteins, quenchers and molocular motors. In an embodiment, the probe is a hydrolysis probe, such as, for example, a TaqMan^{®} probe. In other embodiments, the probes of the invention are molecular beacons, any fluorescent probes, and probes that are replaced by any double stranded DNA binding dyes (*e.g.,* SYBR Green^{®} 1).

Oligonucleotides of the present invention do not only include primers that are useful for conducting the aforementioned amplification reactions, but also include oligonucleotides that are attached to a solid support, such as, for example, a microarray, multiwell plate, column, bead, glass slide, polymeric membrane, glass microfiber, plastic tubes, cellulose, and carbon nanostructures. Hence, detection of CMV and EBV strains can be performed by exposing such a polynucleotide-covered surface to a sample such that the binding of a complementary strain DNA sequence to a surface-attached polynucleotide elicits a detectable signal or reaction.

Oligonucleotides of the present invention also include primers for isolating and sequencing nucleic acid sequences derived from any identified or yet to be isolated and identified CMV and EBV genome.

One embodiment of the invention uses solid support-based oligonucleotide hybridization methods to detect gene expression. Solid support-based methods suitable for praticing the present invention are widely known and are described (PCT application WO 95/11755; Huber et al., Anal. Biochem., 299:24, 2001; Meiyanto et al., Biotechniques, 31:406, 2001, Relogio et al., Nucleic Acids Res., 30:e51, 2002; the contents of which are incorporated herein by reference in their entirety). Any solid surface to which oligonucleotides can be bound, covalently or non-covalently, can be used. Such solid supports include, but are not limited to, filters, polyvinyl chloride dishes, silicon or glass based chips.

In certain embodiments, the nucleic acid molecule can be directly bound to the solid support or bound through a linker arm, which is typically positioned between the nucleic acid sequence and the solid support. A linker arm that increases the distance between the nucleic acid molecule and the substrate can increase hybridization efficiently. There are a number of ways to position a linker arm. In one common approach, the solid support is coated with a polymeric layer that provides linker arms with a plurality of reactive ends/sites. A common example of this type is glass slides coated with polylysine (U.S. Patent No. 5,667,976, the contents of which are incorporated herein by reference in its entirety), which are commercially available. Alternatively, the linker arm can be synthesized as part of or conjugated to the nucleic acid molecule, and then this complex is bonded to the solid support. One approach, for example, takes advantage of the extremely high affinity biotin-streptavidin interaction. The streptavidin-biotinylated reaction is stable enough to withstand stringent washing conditions and is sufficiently stable that it is not cleaved by laser pulses used in some detection systems, such as matrix-assisted laser desorption/ionization time of flight (MALDI-TOF) mass spectrometry. Therefore, streptavidin can be covalently attached to a solid support, and a biotinylated nucleic acid molecule will bind to the streptavidin-coated surface. In one version of this method, an amino-coated silicon wafer is reacted with the *n*-hydroxysuccinimido-ester of biotin and complexed with streptavidin. Biotinylated oligonucleotides are bound to the surface at a concentration of about 20 finol DNA per mm².

Alternatively, one can directly bind DNA to the support using carbodiimides, for example. In one such method, the support is coated with hydrazide groups, then treated with carbodiimide. Carboxy-modified nucleic acid molecules are then coupled to the treated support.. Epoxide-based chemistries are also being employed with amine modified oligonucleotides. Other chemistries for coupling nucleic acid molecules to solid substrates are known to those of skill in the art.

The nucleic acid molecules, *e.g*., the primers and probes of the present invention, must be delivered to the substrate material, which is suspected of containing or is being tested for the presence and number of CMV and/or EBV molecules. Because of the miniaturization of the arrays, delivery techniques must be capable of positioning very small amounts of liquids in very small regions, very close to one another and amenable to automation. Several techniques and devices are available to achieve such delivery. Among these are mechanical mechanisms (*e.g*., arrayers from GeneticMicroSystems, MA, USA) and ink-jet technology. Very fine pipets can also be used.

Other formats are also suitable within the context of this invention. For example, a 96-well format with fixation of the nucleic acids to a nitrocellulose or nylon membrane may also be employed.

After the nucleic acid molecules have been bound to the solid support, it is often useful to block reactive sites on the solid support that are not consumed in binding to the nucleic acid molecule. In the absence of the blocking step, excess primers and/or probes can, to some extent, bind directly to the solid support itself, giving rise to non-specific binding. Non-specific binding can sometimes hinder the ability to detect low levels of specific binding. A variety of effective blocking agents (*e.g*., milk powder, serum albumin or other proteins with free amino groups, polyvinylpyrrolidine) can be used and others are known to those skilled in the art (U.S. Patent No. 5,994,065, the contents of which are incorporated herein by reference in their entirety). The choice depends at least in part upon the binding chemistry.

One embodiment uses oligonucleotide arrays, *e.g*., microarrays, that can be used to simultaneously observe the expression of a number of CMV and EBV strain genes. Oligonucleotide arrays comprise two or more oligonucleotide probes provided on a solid support, wherein each probe occupies a unique location on the support. The location of each probe can be predetermined, such that detection of a detectable signal at a given location is indicative of hybridization to an oligonucleotide probe of a known identity. Each predetermined location can contain more than one molecule of a probe, but each molecule within the predetermined location has an identical sequence. Such predetermined locations are termed features. There can be, for example, from 2, 10, 100, 1,000, 2,000 or 5,000 or more of such features on a single solid support. In one embodiment, each oligonucleotide is located at a unique position on an array at least 2, at least 3 at least 4, at least 5, at least 6, or at least 10 times.

Oligonucleotide probe arrays for detecting gene expression can be made and used according to conventional techniques described (Lockhart et al., Nat. Biotech., 14:1675-1680, 1996; McGall et al., Proc. Natl. Acad. Sci. USA, 93:13555, 1,996; Hughes, et al. Nat. Biotechnol., 19:342,2001), A variety of oligonucleotide array designs are suitable for the practice of this invention.

Generally, a detectable molecule, also referred to herein as a label, can be incorporated or added to an array's probe nucleic acid sequences. Many types of molecules can be used within the context of this invention. Such molecules include, but are not limited to, fluorochromes, chemiluminescent molecules, chromogenic molecules, radioactive molecules, mass spectrometry tags, proteins, and the like. Other labels will be readily apparent to one skilled in the art.

Oligonucleotide probes used in the methods of the present invention, including microarray techniques, can be generated using PCR. PCR primers used in generating the probes are chosen, for example, based on the sequences of Table 2. In one embodiment, oligonucleotide control probes also are used. Exemplary control probes can fall into at least one of three categories referred to herein as (1) normalization controls, (2) expression level controls and (3) negative controls. In microarray methods, one or more of these control probes can be provided on the array with the inventive cell cycle gene-related oligonucleotides.

Normalization controls correct for dye biases, tissue biases, dust, slide irregularities, malformed slide spots, *etc*. Normalization controls are oligonucleotide or other nucleic acid probes that are complementary to labeled reference oligonucleotides or other nucleic acid sequences that are added to the nucleic acid sample to be screened. The signals obtained from the normalization controls, after hybridization, provide a control for variations in hybridization conditions, label intensity, reading efficiency and other factors that can cause the signal of a perfect hybridization to vary between arrays. The normalization controls also allow for the semi-quantification of the signals from other features on the microarray. In one embodiment, signals (*e.g*., fluorescence intensity or radioactivity) read from all other probes used in the method are divided by the signal from the control probes, thereby normalizing the measurements.

Virtually any probe can serve as a normalization control. Hybridization efficiency varies, however, with base composition and probe length. Preferred normalization probes are selected to reflect the average length of the other probes being used, but they also can be selected to cover a range of lengths. Further, the normalization control(s) can be selected to reflect the average base composition of the other probe(s) being used. In one embodiment, only one or a few normalization probes are used, and they are selected such that they hybridize well (*i.e*., without forming secondary structures) and do not match any test probes. In one embodiment, the normalization controls are mammalian genes.

"Negative control" probes are not complementary to any of the test oligonucleotides (*e.g*., the inventive cell cycle gene-related oligonucleotides), normalization controls, or expression controls. In one embodiment, the negative control is a mammalian gene that is not complementary to any other sequence in the sample.

The terms "background" and "background signal intensity" refer to hybridization, signals resulting, from non-specific binding or other interactions between the labeled target nucleic acids (*e.g*., mRNA present in the biological sample) and components of the oligonucleotide array. Background signals also can be produced by intrinsic fluorescence of the array components themselves. A single background signal can be calculated for the entire array, or a different background signal can be calculated for each target nucleic acid. In one embodiment, background is calculated as the average hybridization signal intensity for the lowest 5 to 10 percent of the oligonucleotide probes being used, or, where a different background signal is calculated for each target gene, for the lowest 5 to 10 percent of the probes for each genre. Where the oligonucleotide probes corresponding to a particular CMV and/or EBV target hybridize well and hence, appear to bind specifically to a target sequence, they should not be used in a background signal calculation. Alternatively, background can be calculated as the average hybridization signal intensity produced by hybridization to probes that are not complementary to any sequence found in the sample (*e.g*., probes directed to nucleic acids of the opposite sense or to genes not found in the sample). In microarray methods, background can be calculated as the average signal intensity produced by regions of the array that lack any oligonucleotides probes at all.

In an alternative embodiment, the nucleic acid molecules are directly or indirectly coupled to an enzyme. Following hybridization, a chromogenic substrate is applied and the colored product is detected by a camera, such as a charge-coupled camera. Examples of such enzymes include alkaline phosphatase, horseradish peroxidase and the like. The invention also provides methods of labeling nucleic acid molecules with cleavable mass spectrometry tags (CMST; U.S. Patent No: 60/279,890). After an assay is complete, and the uniquely CMST-labeled probes are distributed across the array, a laser beam is sequentially directed to each member of the array. The light from the laser beam both cleaves the unique tag from the tag-nucleic acid molecule conjugate and volatilizes it. The volatilized tag is directed into a mass spectrometer. Based on the mass spectrum of the tag and knowledge of how the tagged nucleotides were prepared, one can unambiguously identify the nucleic acid molecules to which the tag was attached (WO 9905319).

The nucleic acids, primers and probes of the present invention can be labeled readily by any of a variety of techniques. When the diversity panel is generated by amplification, the nucleic acids can be labeled during the reaction by incorporation of a labeled dNTP or use of labeled amplification primer. If the amplification primers include a promoter for an RNA polymerase, a post-reaction labeling can be achieved by synthesizing RNA in the presence of labeled NTPs. Amplified fragments that were unlabeled during amplification or unamplified nucleic acid molecules can be labeled by one of a number of end labeling techniques or by a transcription method, such as nick-translation, random-primed DNA synthesis. Details of these methods are known to one of skill in the art and are set out in methodology books. Other types of labeling reactions are performed by denaturation of the nucleic acid molecules in the presence of a DNA-binding molecule, such as ReCA, and subsequent hybridization under conditions that favor the formation of a stable RecA-incorporated DNA complex.

In another embodiment, PCR-based methods are used to detect gene expression. These methods include reverse-transcriptase-mediated polymerase chain reaction (RT-PCR) including real-time and endpoint quantitative reverse-transcriptase mediated polymerase chain reaction (Q-RTPCR). These methods are well known in the art. For example, methods of quantitative PCR can be carried out using kits and methods that are commercially available from, for example, Applied BioSystems and Stratagene^{®}, See also Kochanowski, Quantitative PCR Protocols (Humana Press, 1999); Innis *et al*., *supra*.; Vandesompele et al., Genome Biol., 3: RESEARCH 0034, 2002; Stein, Cell Mol. Life Sci. 59:1235, 2002.

The forward and reverse amplification primers and internal hybridization probe is designed to hybridize specifically ang uniquely with one nucleotde sequence derived from the transcript of a target gene. In one embodiment, the selection criteria for primer and probe sequences incorporates constraints regarding nucleotide content and size to accommodate TaqMan^{®} requirements. SYBR Green^{®} can be used as a probe-less Q-RTPCR alternative to the TaqMan^{®}-type assay, discussed above (ABI Prsm® 7900 Sequence Detection System User Guide Applied Biosystems, chap. 1-8, App. A-F. (2002)). A device measures changes in fluorescence emission intensity during PCR amplification. The Measurement is done in "real time," that is, as the amplification product accumulates in the reaction. Other methods can be used to measure changes in fluorescence resulting from probe digestion. For example, fluorescence polarization can distinguish between large and small molecules based on molecular tumbling (U.S. Patent No. 5,593,867).

The primers and probes of the present invention may anneal to or hybridize to various CMV and/or EBV genetic material or genetic material derived thererfom, such as RNA, DNA, cDNA, or a PCR product.

A "sample" that is tested for the presence of CMV and/or EBV strains includes, but is not limited to a tissue sample, such as, for example, blood, serum, plasma, enriched peripheral blood mononuclear cells, bone marrow, urine, neoplastic or other tissue obtained from biopsies, cerebrospinal fluid, saliva, fluids collected from the ear, eye, mouth, and respiratory airways, sputum, urine, stool, skin, semen, seminal fluid, gastric secretions, tears, oropharyngeal swabs, nasopharyngeal swabs, throat swabs, nasal aspirates, nasal wash, renal tissue and fluid therefrom including perfusion media, tissues either to be utilized for transplantation between individuals and/or animal derived tissues to be utilized for transplantation into a human recipient, fluids and cells obtained by the perfusion of tissues of both human and animal origin, and fluids and cells derived from the culturing of human cells, including human stem cells and human cartilage or fibroblasts. The tissue sample may be fresh, fixed, preserved, or frozen. A sample also includes any item, surface, material, or clothing, or environment, for example, sewage or water treatment plants, in which it may be desirable to test for the presence of CMV and/or EBV strains. Thus, for instance, the present invention includes testing door handles, faucets, table surfaces, elevator buttons, chairs, toilet scats, sinks, kitchen children's cribs, bed linen, pillows, keyboards, and so on, for the presence of CMV and/or EBV strains.

The target nucleic acid strain that is amplified may be RNA or DNA or a modification thereof. Thus, the amplifying step can comprise isothermal or non-isothermal reactions, such as polymerase chain reaction, Scorpion^{®} primers, molecular beacons, SimpleProbes^{®}, HyBeacons^{®}, cycling probe technology, Invader Assay, self-sustained sequence replication, nucleic acid sequence-based amplification, ramification amplifying method, hybridization signal amplification method, rolling circle amplification, multiple displacement amplification, thermophilic strands displacement amplification, transcription-mediated amplification, jigase chain reaction, signal mediated amplication of RNA, split promoter amplification, Q-Beta replicase, isothermal chain reaction, one cut event amplification, loop-mediated isothermal amplification, molecular inversion probes, ampliprobe, headloop DNA amplification, and ligation activated transcription. The amplifying step can be conducted on a solid support, such as a multiwell plate, array, column, beard, glass slide, polymeric membrane, glass microfiber, plastic tubes, cellulose, and carbon nanostructures. The amplifying step also comprises *in situ* hybridization. The detecting step can comprise gel electrophoresis, fluorescence resonant energy transfer, or hybridization to a labeled probe, such as a probe labeled with biotin, at least one fluorescent moiety, an antigen, a molecular weight tag, and a modifier of probe Tm. The detection step can also comprise the incorporation of a label (*e.g*., fluorescent or radioactive) during an extension reaction. The detecting step comprises measuring fluorescence, mass, charge, and/or chemiluminescence.

The target nucleic acid strain may not need amplification and may be RNA or DNA or a modification thereof. If amplification is not necessary, the target nucleic acid strain can be denatured to enable hybridization of a probe to the target nucleic acid sequence.

Hybridization may be detected in a variety of way and with a variety of equipment. In general, the methods can be categorized as those that rely upon detectable molecules incorporated into the diversity panels and those that rely upon measurable properties of double-stranded nucleic acids (*e.g.*, hybridized nucleic acids) that distinguish them from single-stranded nucleic acids (*e.g.*, unhybridized nucleic acids). The latter category of methods includes intercalation of dyes, such as, for example, cthidium bromide, into double-stranded nucleic acids, differential absorbance properties of double and single stranded nucleic acids, binding of proteins that preferentially bind double-stranded nucleic acids, and the like.

### EXEMPLIFICATION

### Example 1. Scoring a Set of Predicted Annealing Oligonucleotides

Each of the sets of primers and probes selected is ranked by a combination of methods as individual primers and probes and as a primer/probe set. This involves one or more methods of ranking (*e.g.*, joint ranking, hierarchical ranking, and serial ranking) where sets of primers and probes are eliminated of included based on any combination of the following criteria, and a weighted ranking again based on any combination of the following criteria, for example: (A) Percentage Identity to Target Strains; (B) Conservation Score; (C) Coverage Score; (D) Strain/Subtype/Scrotype Score; (E) Associated Disease Score; (F) Duplicates Sequences Score; (G) Year and Country of Origin Score; (H) Patent Score, and (I) Epidemiology Score.

### (A) Percentage Identity

A percentage identity score is based upon the number of target nucleic acid strain (*e.g.*, native) sequences that can hybridize with perfect conservation (the sequences are perfectly complimentary) to each primer or probe of a primer set and probe set. If the score is less than 100%, the program ranks additional printer set and probe sets that are not perfectly conserved. This is a hierarchical scale for percent identity starting with perfect complimentarity, then one base degeneracy through to the number of degenerate bases that would provide the score closet to 100%. The position of these degenerate bases would then be ranked. The methods for calculating the conservation is described under section B.

### (i) Individual Base Conservation Score

A set of conservation scores is generated for each nucleotide base in the consensus sequence and these scores represent how many of the target nucleic acid strains sequences have a particular base at this position. For example, a score of 0.95 for a nucleotide with an adenosine, and 0.05 for a nucleotide with a cytidine means that 95% of the native sequences have an A at that position and 5% have a C at that position. A perfectly conserved base position is one where all the target nucleic acid strain sequences have the same base (either an) A, C, G, or T/U) at that, position, If there is an equal number of bases (*e.g.*, 50% A & 50% T) at a position, it is identified with an N.

### (ii) Candidate Primer/Probe Sequence Conservation

An overall conservation score is generated for each candidate primer or probe sequence that represents how many of the target nucleic acid strain sequences will hybridize to the primers or probes. A candidate sequence that is perfectly complimentary to all the target nucleic acid strain sequences will have a score of 10 and rank the higest. For example, illustated below in Table 1 are different 10-base candidate probe sequences that are targeted to different regions of a consensus target nucleic acid strain sequence. Each candidate probe sequence is compared to a total of 10 native sequences.

**Table 1**

| #1. | A | A | A | C | A | C | G | T | G | C |
|---|---|---|---|---|---|---|---|---|---|---|
| | 0.7 | 1.0 | 1.0 | 1.0 | 1.0 | 1.0 | 1.0 | 1.0 | 1.0 | 1.0 |
| SEQ ID NO: 176 | | | | | | | | | | |
| →Number of target nucleic acid strain sequences that are perfectly complimentary - 7. Three out of the ten sequences do not have an A at position 1. | | | | | | | | | | |

| #2. | C | C | T | T | G | T | T | C | C | A |
|---|---|---|---|---|---|---|---|---|---|---|
| | 1.0 | 0.9 | 1.0 | 0.9 | 0.9 | 1.0 | 1.0 | 1.0 | 1.0 | 1.0 |
| SEQ ID NO: 177 | | | | | | | | | | |
| →Number of target nucleic acid strain sequences that are perfectly complimentary - 7, 8, or 9 At least one target nucleic acid strain does not have a C at position 2, T at position 4, or G at position 5. These differences may all be on one target nucleic acid strain molecule or may be on two or three separate molecules. | | | | | | | | | | |

| #3. | C | A | G | G | G | A | C | G | A | T |
|---|---|---|---|---|---|---|---|---|---|---|
| | 1.0 | 1.0 | 1.0 | 1.0 | 1.0 | 0.9 | 0.8 | 1.0 | 1.0 | 1.0 |
| SEQ ID NO: 178 | | | | | | | | | | |
| →Number of target nucleic acid strain sequences that are perfectly complimentary -7 or 8. At least one target nucleic acid strain does not have an A at position 6 and at least two target nucleic acid strain do not have a C at position 7. These differences may all be on one target nucleic acid strain molecule or may be on two separate molecules. | | | | | | | | | | |

A simple arithmetic mean for each candidate sequence would generate the same value of 0.97. The number of target nucleic acid strain sequences identified by each candidate probe sequence, however, can be very different. Sequence #1 can only identify 7 native sequences because of the 0.7 (out of 1.0) score by the first base - A. Sequence #2 has three bases each with a score of 0.9; each of these could represent a different or shared target nucleic acid strain sequence. Consequently, Sequence #2 can identify 7, 8 or 9 target nucleic acid strain sequences. Similarly, Sequence #3 can identify 7 or 8 of the target nucleic acid strain sequences. Sequence #2 would, therefore, be the best choice it all the three bases with a score of 0.9 represented the same 9 target nucleic acid strain sequences.

### (iii) Overall Conservation Score of the Primer and Probe Set-Percent Identity

The same method described in (ii) when applied to the complete primer set and probe set will generate the percent identity for the set (see A above). For example, using the same sequences illustrated above, if Sequences #1 and #2 are primers and Sequence #3 is a probe, then the percent identity for the target can be calculated from how many of the target nucleic acid strain sequences are identified with perfect complimentarity by all three primer/probe sequences. The percent identity could be no better than 0.7 (7 out of 10 target nucleic acid strain sequences) but as little as 0.1 if each of the degenerate bases reflects a different target nucleic acid strain sequence. Again, an arithmetic mean of these three sequences would be 0.97. As none of the above examples were able to capture all the target nucleic acid strain sequences because of the degeneracy (scores of less than 1.0), the ranking system takes into account that a certain amount of degeneracy can be tolerated under normal hybridization conditions, for example, during a polymerase chain reaction. The ranking of these degeneracies is described in (iv) below.

An *in silico* evaluation determines how many native sequences (*e.g.*, onginal sequences submitted to public databases) are identified by a given candidate primer/probe set. The ideal candidate primer/probe set is one that can perform PCR and the sequences are perfectly complimentary to all the known native sequences that were used to generate the consensus sequence. If there is no such candidate, then the sets are ranked according to how many degenerate bases can be accepted and still hybridize to just the target sequence during the PCR and yet identity all the native sequences.

The hybridization conditions, for TaqMan^{®} as an example, are: 10-50 mM Tris-HCl pH 8.3, 50 mM KCl, 0.1-0.2% Triton^{®} X-100 or 0.1% Tween^{®}, 1-5mM MgCl₂. The hybridization is performed at 58-61°C for the primers and 68-70°C for the probe. The in silico PCR identifies native sequences that are not amplifiable using the candidate primers and probe set. The rules can be as simple as counting the number of degenerate bases to more sophisticated approaches based on exploiting the PCR criteria used by the PriMD^{®} software. Each target nuclelic acid strain sequence has a value or weight (see Score assignment above). If the failed target nueleic acid strain sequences is medically valuable, the primer/probe set is rejected. This in silico analysis provides a degree of confidence for a given genotype and is important when new sequences are added to the databases. New target nucleic acid strain sequences are automatically entered into both the include" and "exclude" categories. Published primer and probes will also be ranked by the PriMD sofware.

### (iv) Position (5' to 3') Of The Base Conservation Score

In an embodiment, primers do not have bases in the terminal five positions at the end with a score less than 1. This is one of the last parameters to be relaxed if the method fails to select any candidate sequences. The next best candidate having a perfectly conserved primer would be one where the poorer conserved positions are limited to the terminal bases at the 5' end. The closer the poorer conserved position is to the 5' end, the better the score, For probes, the position criteria is different. For example, with a TaqMan^{®} probe, the most destabilizing effect occurs in the center of the probe. The 5' end of the probe is also important as this contains the reporter molecule that must be cleaved, following hybridization to the target, by the polymerase to generate a sequence-specific signal. The 3' end is less critical. Therefore, a sequence with a perfectly conserved middle region will have the higher score, The remaining ends of the probe are ranked in a similar fashion to the 5' end of the primer. Thus, the next best candidate to a perfectly conserved TaqMan^{®} probe would be one where the poorer conserved positions are limited to the terminal bases at either the 5' or 3' ends. The hierarchical scoring will select primers with only one degeneracy first, then primers with two degeneracies next and so on. The relative position of each degeneracy will then be ranked favoring those that are closest to the 5' end of the primers and those closes to the 3' end of the TaqMan^{®} probe. If there are two or more degenerate bases in a primer and probe set the tankíng will initially select the sets where the degeneracies occur on different sequences.

### B. Coverage Score

The total number of aligned sequences is considered under a coverage score. A value is assigned to each position based on how many times that position has been reported or sequenced. Alternatively, coverage can be defined as, how representative the sequences are of known strains, subtypes etc., or their relevance to a certain diseases. For exemple, the target nucleic acid strain sequences for a particular gene may be very well conserved and show complete coverage but certain strains are not represented in those sequences.

A sequenceis included if it aligns with any part of the consensus sequence, which is usually a whole gene or a functional unit, or has been described as being a representative of this gene. Even though a base position is perfectly censerved it may only represent a fraction of the total number of sequences (for example, if there are very few sequences). For example, region A of a gene shows a 100% conservation from 20 sequence entires while region B in the same gene shows a 98% conservation but from 200 sequence entries. There is a relationship between conservation and coverage if the sequence shows some persistent variabily. As more sequences are aligned, the conservation score falls, but this effect is lessened as the number of sequences gets larger. Unless the number of sequences is very small (*e.g.*, under 10) the value of the coverage score is small compared to that of the conservation score. To obtain the best consensus sequence, artificial spaces are allowed to be introduced. Such spaces are not considered in the coverage score.

### C. Strain/Subtype/Serotype Score

A value is assigned to each stain or subtype or serotype based upon its relevance to a disease. For example, strains of CMV and/or EBV that are linked to high frequencies of infection will have a higher score than strains that are generally regarded as benign. The score is based upon sufficient evidence to automatically associate a particular strain with a disease. For example, certain strains of adenovirus are not associated with diseases of the upper respiratory system. Accordingly, there will be sequences included in the consensus sequence that are not associated with diseases of the upper respiratory system.

### D. Associated Disease Score

The associated disease score pertains to strains that are not known to be associated with a particular disease (to differentiate from D above). Here, a value is assigned only if the submitted sequence is directly linked to the disease and that disease is pertinent to the assay.

### E. Duplicate Sequences Score

If a particular sequence has been sequenced more than once it will have an effect on representation, for example, a strain that is represented by 12 entries in GenBank of which six are identical and the other six are unique. Unless the identical sequences can be assigned to different strains/subtypes (usually by sequencing other gene or by immunology methods) they will be excluded from the scoring.

### F. Year and Country of Origin Score

The year and country of origin scores are important in terms of the age of the human population and the need to provide a product for a global market. For example, strains identified or collected many years ago may not be relevant today. Furthermore, it is probably difficult to obtain samples that contain these older strains. Certain divergent strains from more obscure countries or sources may also be less relevant to the locations that will likely perform clinical tests, or may be more important for certain countries (*e.g.*, North America, Europe, or Asia).

### G. Patent Score

Candidate target strain sequences published in patents are searched electronically and annotated such that patented regions are excluded. Alternatively, candidate sequences are checked against a patented sequence database.

### H. Minimum Qualifying Score

The minimum qualifying score is determined by expanding the number of allowed mismatches in each set of candidate primers and probes until all possible native sequences are represented (*e.g.*, has a qualifying hit).

### I. Other

A score is given to based on other parameters, such as relevance to certain patients (*e.g.*, pediatries, immunocompromised) or certain therapies (*e.g.*, target those strains that respond to treatment) or epidemiology. The prevalence of an organism/strain and the number of times it has been tested for in the community can add value to the selection of the candidate sequences. If a particular strain is more commonly tested then selection of it would be more likely. Strain identification can be used to select better vaccines.

### Example 2. Primer/Probe Evaluation

Once the candidate primers and probes have received their scores and have been ranked, they are evaluated using any of a number of methods of the invention, such as BLAST analysis and secondary structure analysis.

### A. BLAST Analysis

The candidate primer/probe sets are submitted to BLAST analysis to check for possible overlap with any published sequences that might he missed by the Include/Exclude function. It also provides a useful summary.

### B. Secondary Structure

The methods of the present invention include analysis of nucleic acid secondary structure. This includes the structures of the primers and/or probes, as well as their intended target strain sequences. The methods and software of the invention predict the optimal temperatures for annealing but assumes that the target (*e.g.*, RNA or DNA) does not have any significant secondary structure. For example, if the starting material is RNA, the first stage is the creation of a complimentary strand of DNA (cDNA) using a specific primer. This is usually performed at temperatures, where the RNA template can have significant secondary structure thereby preventing the annealing of the primer. Similarly, after denaturation of a double stranded DNA target (for example, an amplicon after PCR), the binding of the probe is dependent on there being no major secondary structure in the amplicon.

The methods of the invention can either use this information as a criteria for selecting primers and probes or evaluate any secondary structure of a selected sequence, for example, by cutting and pasting candidate primer or probe sequences into a commercial internet link that uses software dedicated to analyzing secondary structure, such as, for example, MFOLD (Zuker et al. (1999) Algorithms and Thermodynamics for RNA Secondary Structure Prediction: A Practical Guide in RNA Biochemistry and Biotechnology, J. Barciszewski and B.F.C. Clark, eds., NATO ASI Series,Kluwer Academic Publishers).

### C. Evaluating the Primer and Probe Sequences

The methods and software of the invention may also analyze any nucleic acid sequence to determine its suitability in a nucleic acid amplification-based assay. For example, it can accept a competitor's primer set and determine the following information. (1) How it compares to the primers of the invention (*e.g.*, overall rank, PCR and conservation ranking, etc.); (2) How it aligns to the exclude libraries (*e.g.*, assessing cross-hybridization) - also used to compare primer and probe sets to newly published sequences; and (3) If the sequence has been previously published. This step requires keeping a database of sequences published in scientific journals, posters, and other presentations.

### Example 3. Multiplexing

The Exclude/Include capability is ideally suited for designing multiplex reactions. The parameters designing multiple primer and probe sets adhere to a more stringent set of parameters than those used for the initial Exclude/Include function. Each set of primers and probe, together with the resulting amplicon, is screened against the other sets that constitute the multiplex reaction. As new target are accepted, their sequences are automatically added to the Exclude category.

The database is designed to interrogate the online databases to determine and acquire, if necessary, any new sequences relevant to the targets. These sequences are evaluated against the optimal primer/probe set. If they represent a new genotype or strain, then a multiple sequence alignment may be required.

### Example 4. Sequences identified for Detecting CMV and EBV

The set of primers and probes were then scored according to the methods described herein to identify the optimized primers and probes of Table 2. The internal control plasmid and CMV/EBV positive control plasmid are listed in Table 3. A non-competitive control may also be used.

It should be noted that the primers, as they are sequences that anneal to a plurality of all identified of unidentified CMV and EBV strains, can also be used as probes either in the presence or absence of amplification of a sample.

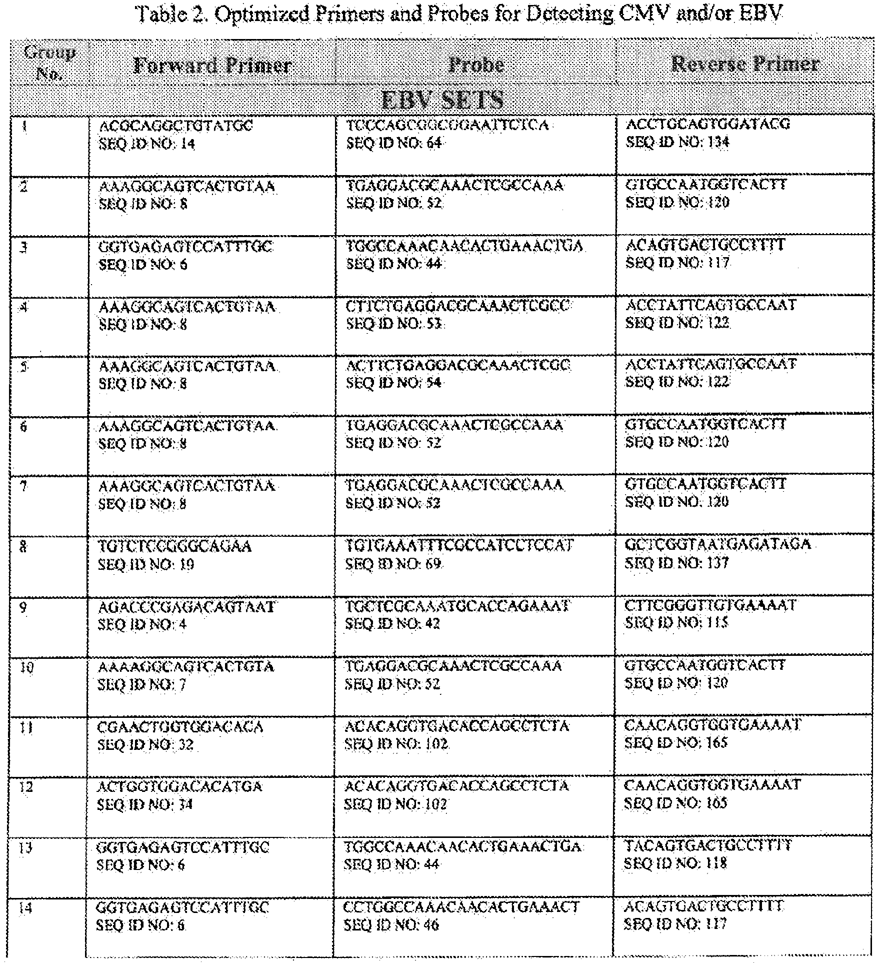

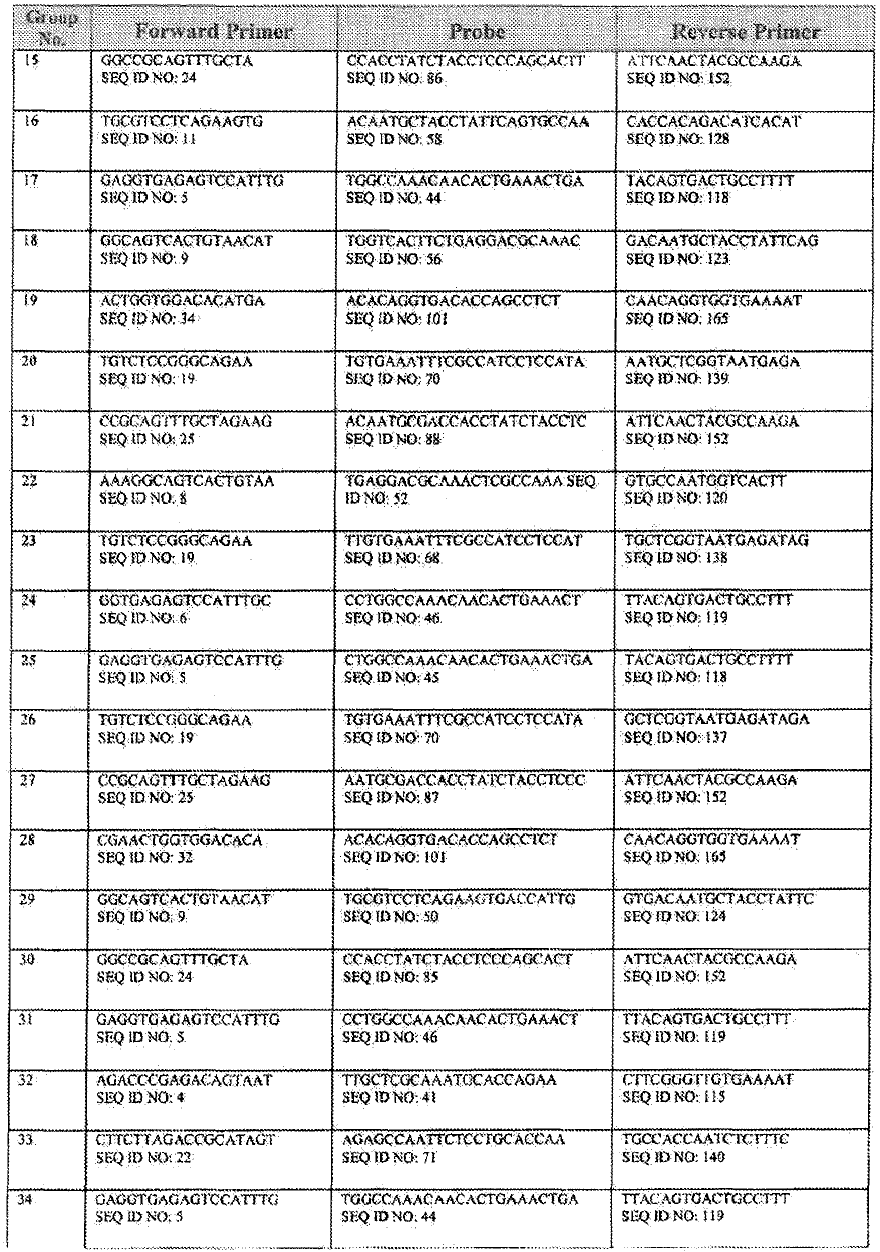

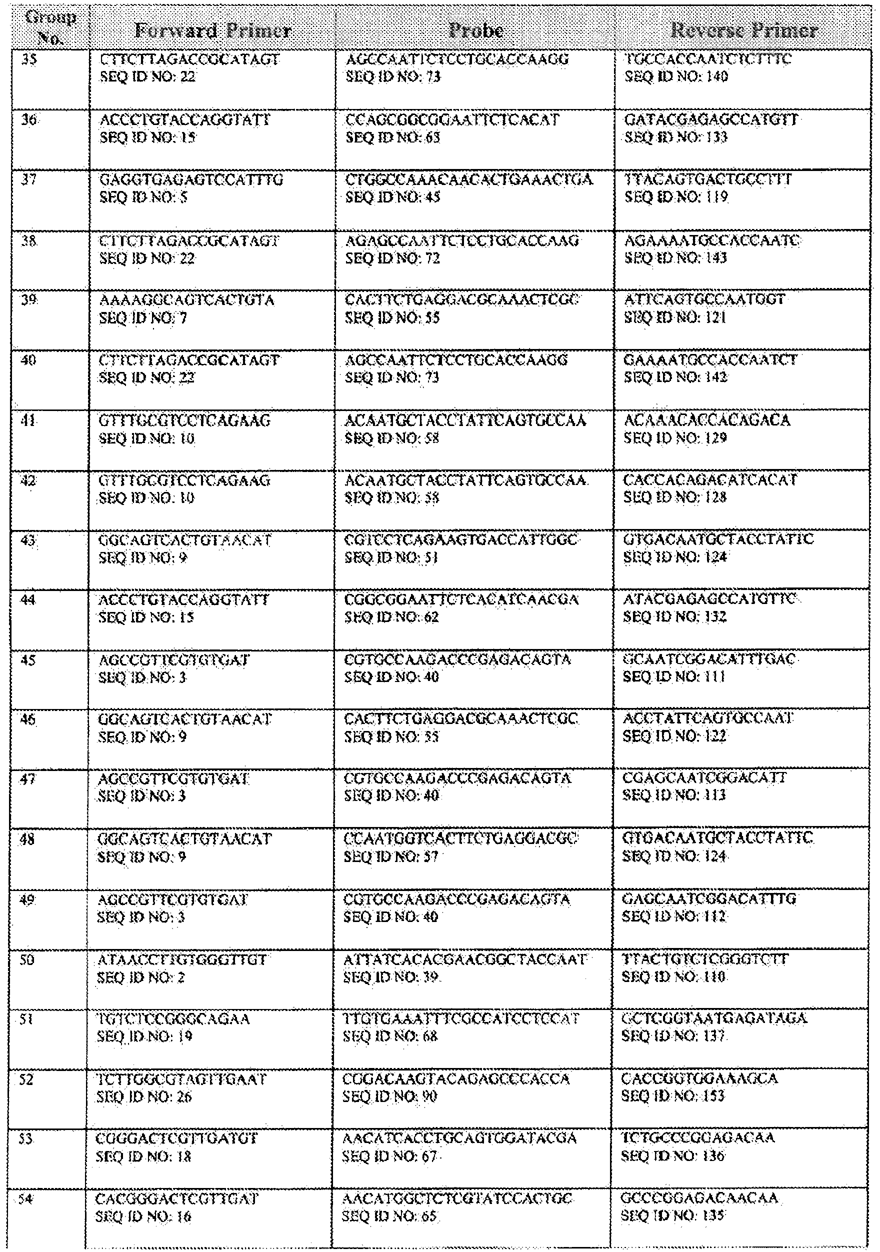

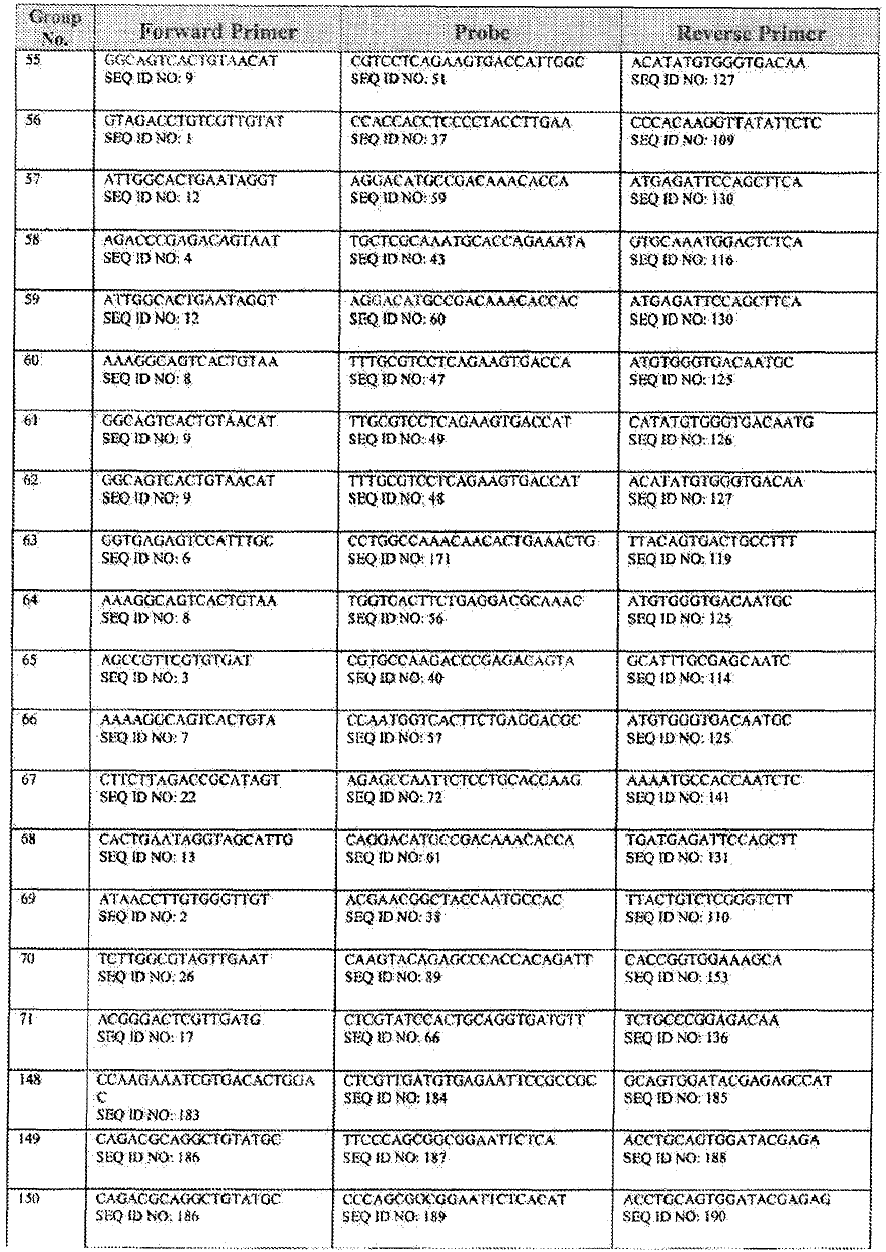

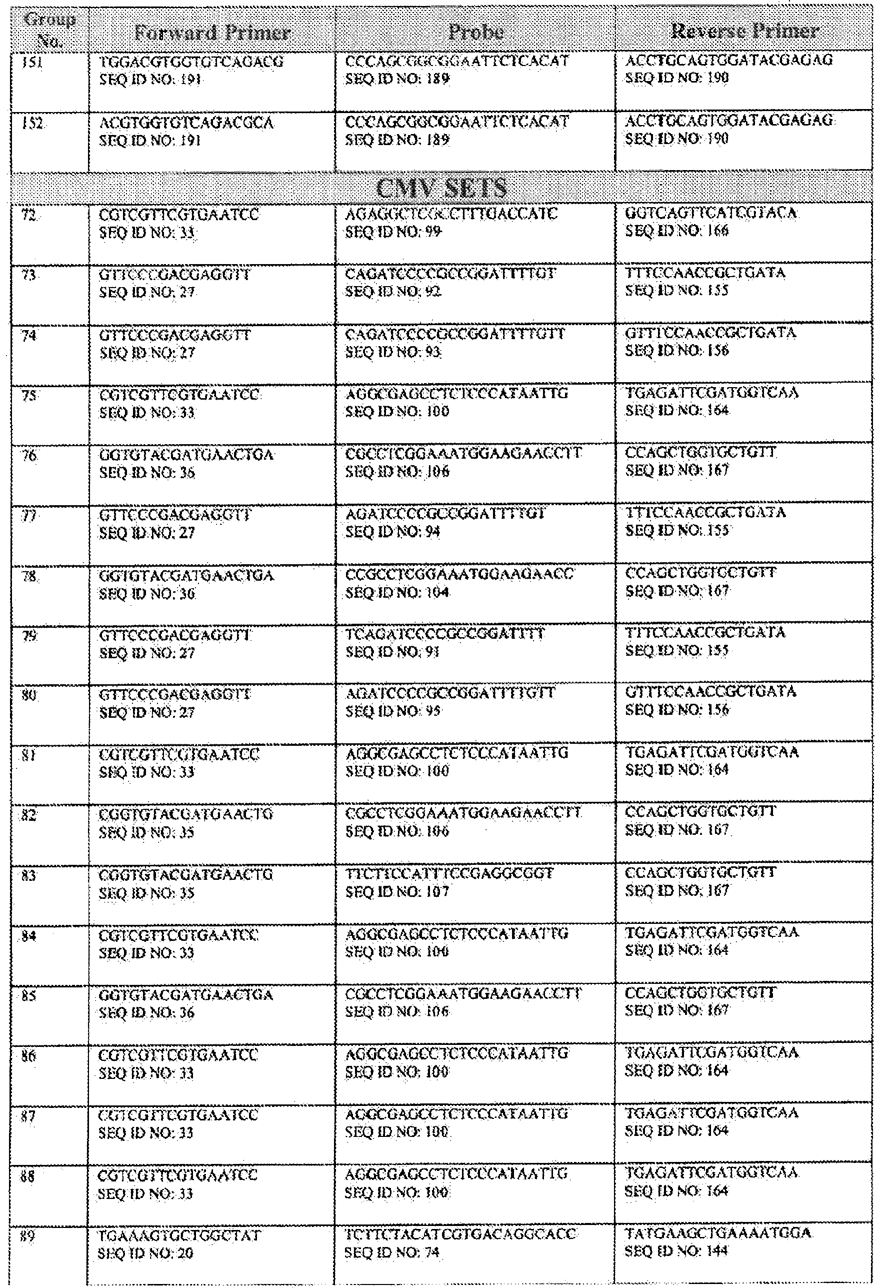

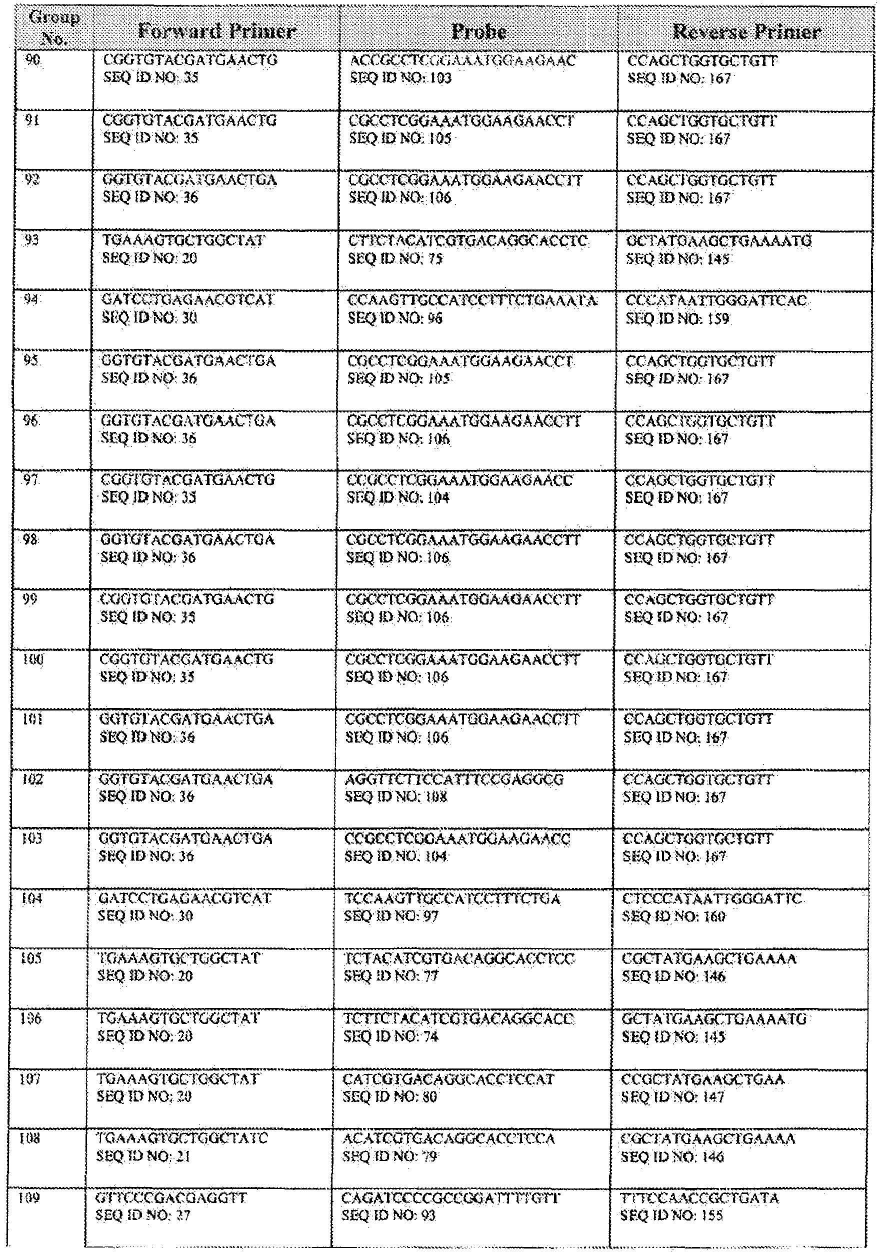

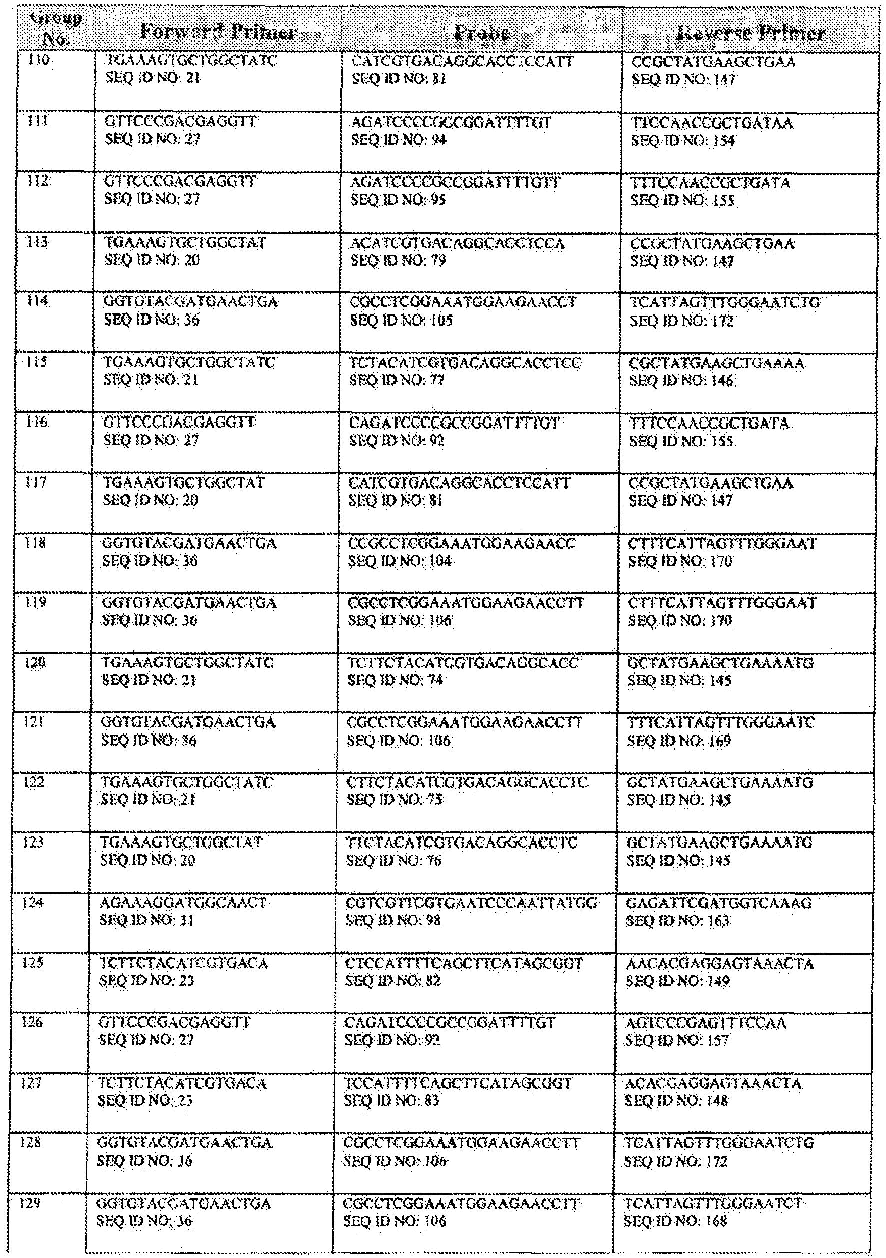

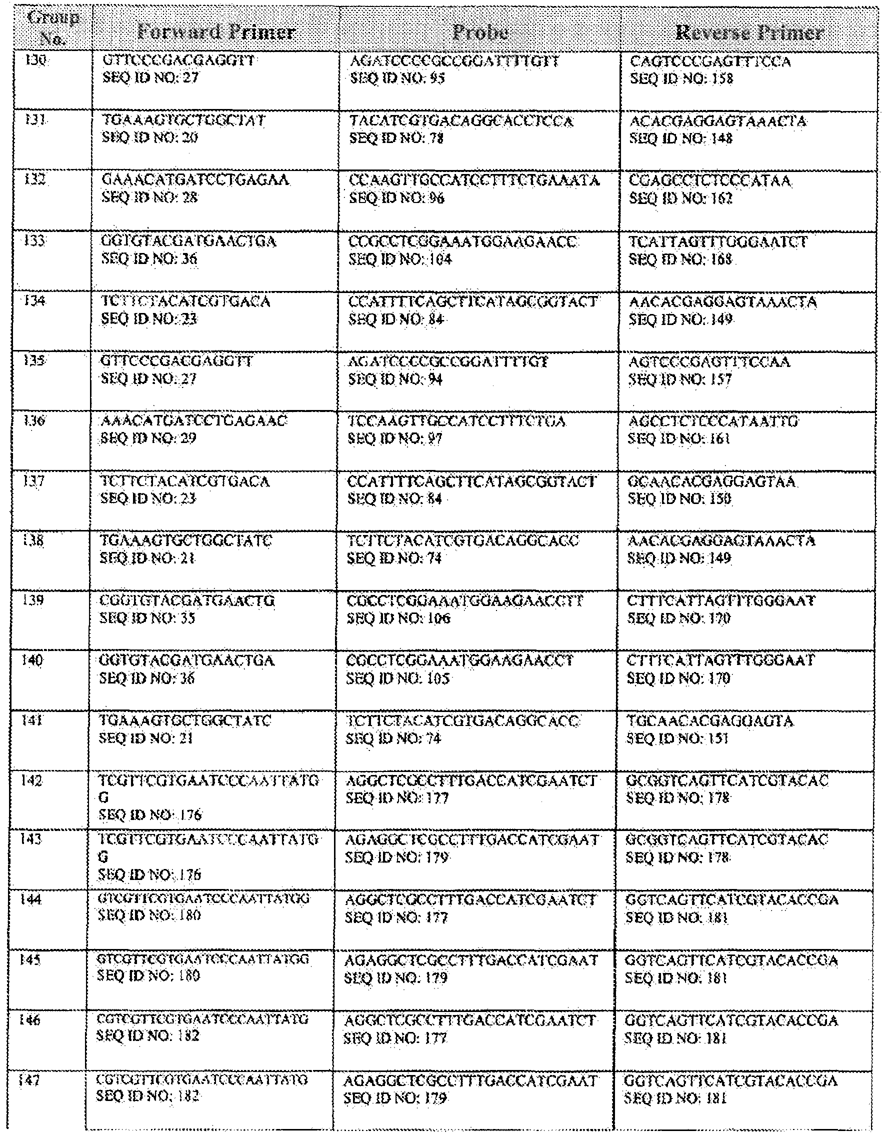

**Table 3. Internal Control Plasmids and Probes for Detecting CMV and/or EBV**

| **Plasmid** | **Target Sequence** | **Probe** | **Primer set** |
|---|---|---|---|
| **Internal Control Plasmid** | 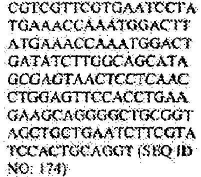 | 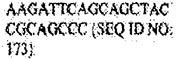 | CGTCGTTCGTGAATCC (SEQ ID NO:33) CMV Forward Primer |
| (Synthetic target sequence (SEQ ID NO 174), cloned into a pUC119-derived vector; plasmid sequence data not provided) | | | ACCTGCAGTGGATACG (SEQ ID NO:134) EBV Reverse Primer |
| **CMV/EBV Positive Control Plasmid** | 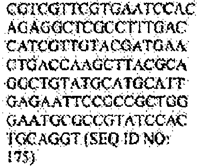 | 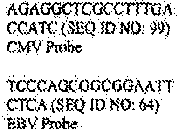 | CGTCGTTCGTGAATCC (SEQ ID NO:33) CMV Forward Primer |
| (Target sequence (SEQ ID NO: 175) cloned into a pUC119-derived vector;plasmid sequence data not provided) | | | GGTCAGTTCATCGTACA (SEQ ID NO. 166) CMV Reverse Primer |
| | | | ACGCAGGCTGTATCG (SEQ ID NO: 14) EBV Forward Primer |
| | | | ACCTGCAGTGGATACG (SEQ ID NO: 134) EBV Reverse Primer |

A PCR primer set for amplifying CMV DNA comprises at least one of the following sets of primer sequences:(1) SEQ ID NOS: 3 3 and 166; (2) SEQ ID NOS: 27 and 155; (3) SEQ ID NOS: 27 and 156; (4) SEQ ID NOS: 33 and 164; (5) SEQ ID NOS: 36 and 167; (6) SEQ ID NOS: 20 and 144; (7) SEQ ID NOS: 20 and 145; (8) SEQ ID NOS: 30 and 159; (9) SEQ ID NOS: 35 and 167; (10) SEQ ID NOS: 30 and 160; (11) SEQ ID NOS: 20 and 146; (12) SEQ ID NOS: 20 and 147; (13) SEQ ID NOS:21 and 146; (14) SEQ ID NOS: 27 and 154; (15) SEQ ID NOS: 21 and *147; (16) SEQ ID NOS: 36 and 172; (17) SEQ ID NOS: 36 and 170; (18) SEQ ID NOS: 21 and 145; (19) SEQ ID NOS: 36 and 169; (20) SEQ ID NOS: 31 and 163; (21) SEQ ID NOS: 23 and 149; (22) SEQ ID NOS: 27 and 157; (23) SEQ ID NOS: 23 and 148; (24) SEQ ID NOS: 36 and 168; (25) SEQ ID NOS: 27 and 158; (26) SEQ ID NOS: 20 and 148; (27) SEQ ID NOS:28 and 162; (28) SEQ ID NOS: 29 and 161; (29) SEQ ID NOS: 23 and 150; (30) SEQ ID NOS: 21 and 149; (31) SEQ ID NOS: 35 and 170; (32) SEQ ID NOS:21 and 151; (33) SEQ ID NOS: 176 and 178; (34) SEQ ID NOS: 180 and 181; and (35) SEQ ID NOS: 182 and 181. Any set of primers can be used simultaneously in a multiplex reaction with one or more other primer sets, so that multiple amplicons are amplified simultaneously.

The preceding numbering of the thirty-five sets of primers does not correspond exactly to the "Group" numbering scheme in Table 2 because certain groups use the same primer set, but different internal probes. For example, Groups 73, 77, 79, 109, 112 and 116 of Table 2 each employ the forward primer of SEQ ID NO:27 and the reverse primer of SEQ ID NO: 155, but different internal probes in each instance, *e.g.,* SEQ ID NOS: 92,94,91, 93,95, and 92. Accordingly, primer set "(2)" of the preceding passage implies any one of Groups 73,77,79,109,112 and 116 of Table 2.

A probe for binding to CMV DNA comprisess at least one of the following probe sequences: SEQ ID NOS: 74-84, 91,-100, 103-108, 177 and 179.

A PCR primer set for amplifying EBV DNA comprises at least one of the following sets of primer sequences:(1) SEQ ID NOS: 14 and 134; (2) SEQ ID NOS:8 and 120; (3) SEQ ID NOS; 6 and 117; (4) SEQ ID NOS:8 and 122; (5) SEQ ID NOS: 8 and 120; (6) SEQ ID NOS: 19 and 137; (7) SEQ ID NOS:4 and 115; (8) SEQ ID NOS:7 and 120; (9) SEQ ID NOS:32 and 165; (10) SEQ ID NOS: 34 and 165; (11) SEQ ID NOS: 6 and 118; (12) SEQ ID NOS: 24 and 152; (13) SEQ ID NOS: 11 and 128; (14) SEQ ID NOS: 5 and 118; (15) SEQ ID NOS:9 and 123;(16) SEQ ID NOS: 19 and 139; (17) SEQ ID NOS 6 and 119: (18) SEQ ID NOS:25 and 152; (19) SEQ ID NOS: 19 and 138; (20) SEQ ID NOS: 9 and 124; (21) SEQ ID NOS: 5 and 119; (22) SEQ ID NOS: 22 and 140; (23) SEQ ID NOS: 15 and 133; (24) SEQ ID NOS: 22 and 143; (25) SEQ ID NOS: 7 and 121; (26) SEQ ID NOS: 22 and 142; (27) SEQ ID NOS: 10 and 129; (28) SEQ ID NOS: 10 and 128; (29) 15 and 132; (30) SEQ ID NOS: 3 and 111; (31) SEQ ID NOS: 9 and 122; (32) SEQ ID NOS: 3 and 113; (33) SEQ ID NOS: 3 and 112; (34) SEQ ID NOS:2 and 110; (35) SEQ ID NOS: 26 and 153; (36) SEQ ID NOS: 18 and 136; (37) SEQ ID NOS: 16 and 135; (38) 9 and 127; (39) SEQ ID NOS: 1 and 109; (40) SEQ ID NOS: 12 and 130; (41) SEQ ID NOS: 4 and 116; (42) SEQ ID NOS: 8 and 125; (43) SEQ ID NOS: 9 and 126; (44) SEQ ID NOS: 3 and 114; (45) SEQ ID NOS: 7 and 125; (46) SEQ ID NOS: 22 and 141; (47) SEQ ID NOS: 13 and 131; (48) SEQ ID NOS: 17 and 136; (49) SEQ ID NOS: 183 and 185; (50) SEQ ID NOS: 186 and 188; (51) SEQ ID NOS: 186 and 190; and (52) SEQ ID NOS: 191 and 190 Any set of primers can be used simultaneously in a multiplex reaction with one or more other primer sets, so that multiple amplicons are amplified simultaneously.

The preceding numbering of the fifty-two sets of primers does not correspond exactly to the "Group" numbering scheme in Table 2 because certain groups use the same primer set, but different internal probes. For example, Groups 4 and 5 of Table 2 each employ the forward primer of SEQ ID NO: 8 and the reverse primer of SEQ ID NO: 122, but different internal probes, *e.g*., SEQ ID NOS:53 and 54. Accordingly, primer set "(4)" of the preceding passage implies any one of Groups 4 and 5 of Table 2.

A probe for binding to EBV DNA comprises at least one of the following probe sequences: SEQ ID NOS: 37-73, 85-90, 101, 102, 171, 184, 187 and 189.

A multiplex set of primer sets for amplifying CMV and EBV DNA simultaneously comprises a nucleotide sequence selected from the primer sets consisting of: Groups 1-152 of Table 2. A multiplex of polynucleotide probes for binding to CMV and EBV DNA comprises a nucleotide sequence selected from the group consisting of: SEQ ID NOS: 74-84, 91-100, 103-108, 177, 179 (CMV probes) and 37-13, 85-90, 101, 102, 171, 184, 187 and 189. (EBV probes).

An internal contol plasmid employs a single primer from each of the CMV and EBV primer sets (SEQ ID NOS:33 and 134, respectively). The internal control probe (SEQ ID NO:173) is a unique probe that recognizes the synthetic target sequence (SEQ ID NO:174). The plasmid vector containing the internal control target sequence and its specific probe (SEQ ID NO: 173) are included in the assay. The internal control plasmid may either be added directly to the reaction mix to monitor PCR efficiency or to the sample for use as a process control, to monitor efficiency of both sample extraction and PCR.

The CMV/EBV positive control plasmid contain a partial amplicon sequence for both CMV and EBV. The positive control plasmid comprises the forward primer, probe and reverse primer sequences for CMV (SEQ ID NOS: 33, 99 and 166, respectively), followed by a HindIII restriction site, and the forward primer, probe and reverse primer sequences for EBV (SEQ ID NOS: 14, 64 and 134, respectively). The CMV/EBV positive control plasmid may be used to generate a standard curve to quantitate the amount of CMV and EBV DNA present in a reaction or may be used to confirm that the assay is performing to specifications. A non-competitive internal control may also be used. Example 5.

To demonstrate functionality of the PriMD^{®} -generated designs, representative primer-probe sets for CMV and EBV were each tested by real-time PCR on genomic DNA and isolated from their respective organisms using the Applied Biosystems 7500 RT PCR system. Sets were tested individually (singleplex format) and in combination (multiplex format). To enable discrimination and detection of CMV and EBV, the probes for the two targets were differentially labeled: FAM for CMV and Quasar 670 for EBV. PCR was carried out using the following protocol: hot-start activation for 3 minutes at 95°C, followed by 40 cycles of 2-temperature PCR, alternating between 15 seconds at 95°C and 1 minute at 60°C. Fluorescence generated from cleavage of the probes was then plotted over the 40 cycles to generate amplification profiles of CMV and EBV. The results generated for each target in the multiplex format are comparable to those observed with the individual sets in singleplex format, which demonstrates validity of the PriMD^{®} multiplex design process. FIG.1 shows the results of using representative EBV and CMV primer-probe sets to detect the respective virus in either singleplex or multiplex sets.

### Other Embodiments

Other embodiments will be evident to those of skill in the art. It should be understood that the foregoing detailed description is provided for clarity only and is merely exemplary. All references are incorporated herein by reference in their entireties. The spirit and scope of the present invention are not limited to the above examples, but are encompassed by the following claims.

The invention can be further described with reference to the following clauses:
1. An isolated nucleic acid sequence comprising a sequence selected from the group consisting of: SEQ ID NOS: 1-191.
2. A method of hybridizing one or more isolated nucleic acid sequence comprising a sequence selected from the group consisting of: SEQ ID NOS: 1 -172 and 176-191 to a CMV or HBV sequence, comprising contacting one or more isolated nucleic acid sequences to a sample comprising the CMV or EBV sequence under conditions suitable for hybridization.
3. The method of Clause 2, wherein the CMV or EBV sequence is a genomic sequence, a template sequence or a sequence derived from an artificial construct.
4. The method of Clause 2, further comprising isolating the hybridized CMV and/or EBV sequence.
5. The method of Clause 2, further comprising sequencing the hybridized CMV and/or EBV sequence.
6. A primer set comprising at least one forward primer selected from the group consisting of SEQ ID NOS: 1 -36, 176, 180, 182, 183,186 and 191 and at least one reverse primer selected from the group consisting of SEQ ID NOS: 109-170, 172, 178, 181, 185, 188 and 190.
7. The primer set of Clause 6, wherein at least one primer set of Clause 7, wherein the primer set is selected from the group consisting of: Groups 1 -152 of Table 2.
8. A method of producing a nucleic acid product, comprising contacting one or more isolated nucleic acid sequences to a sample comprising the CMV or EBV sequence under conditions suitable for nucleic acid polymerization.
9. The method of Clause 5, wherein the nucleic acid product is an amplicon produced using at least one forward primer selected from the group consisting of SEQ ID NOS: 1-36, 176, 180, 182, 183, 186 and 191 and at least one reverse primer selected from the group consisting of SEQ ID NOS: 109-170, 172, 178, 181, 185, 188 and 190.
10. A probe that hybridizes to the nucleic acid product of Clause 8.
11. The probe of Clause 9, wherein the probe comprises a sequence selected from the group consisting of: SEQ ID NOS: 37-108, 171, 177, 179, 184, 187 and 189.
12. The probe of Clause 10, wherein the probe is labeled with a detectable label selected from the group consisting of: a fluorescent label, a chemiluminescent label, a quencher, a radioactive label, biotin and gold.
13. A set of probes that hybridize to the amplicon of Clause 5, wherein a first probe comprises a sequence selected from the group consisting of: SEQ ID NOS: 74-84, 91-100, 103-108, 177 and 179 and a second probe comprises a sequence selected from the group consisting of: SEQ ID NOS: 37-73, 85-90, 101, 102, 171, 184, 187 and 189.
14. The set of probes of Clause 13, wherein the first probe is labeled with a first detectable label and the second probe is labeled with a second detectable label.
15. The set of probes of Clause 13, wherein the first probe and the second probe are labeled with the same detectable label.
16. The set of probes of Clause 14, wherein the detectable labels are selected from the group consisting of: a fluorescent label, a chemiluminescent label, a quencher, a radioactive label, biotin and gold.
17. A method for detecting and/or screening and/or quantitating CMV or EBV in a sample, comprising:
   a) contacting the sample with at least one forward primer comprising a sequence selected from the group consisting of SEQ ID NOS: 1-36, 176, 180, 182, 183, 186 and 191 and at least one reverse primer comprising a sequence selected from the group consisting of: SEQ ID NOS: 109-170, 172, 178, 181, 185, 188 and 190 under conditions such that nucleic acid amplification occurs to yield an amplicon; and
   b) contacting the amplicon with one or more probes comprising one or more sequences selected from the group consisting of: SEQ ID NOS: 37-108, 171, 177. 179, 184, 187 and 189 under conditions such that hybridization of the probe to the amplicon occurs;
      wherein the hybridization of the probe is indicative of CMV and/or EBV in the sample, and wherein the extent of hybridization is optionally quantified.
18. The method of Clause 17, wherein each of the one or more probes is labeled with a different detectable label.
19. The method of Clause 17, wherein the one or more probes are labeled with the same detectable label.
20. The method of Clause 17, wherein the sample is selected from the group consisting of: blood; serum; plasma; enriched peripheral blood mononuclear cells; bone marrow; urine; neoplastic or other tissue obtained from biopsies; cerebrospinal fluid; saliva; fluids collected from the car, eye, mouth and respiratory airways; sputum; urine; stool; skin; semen; seminal fluid; gastric secretions; tears; oropharyngeal swabs; nasopharyngeal swabs; throat swabs; nasal aspirates; nasal wash; renal tissue and fluid therefrom including perfusion media; tissues either to be utilized for transplantation between individuals or animal-derived tissues to be utilized for transplantation into a human recipient; fluids and cells obtained by the perfusion of tissues of both human and animal origin; and fluids and cells derived from the culturing of human cells, human stem cells, human cartilage or fibroblasts.
21. The method of Clause 17, wherein the sample is from a human.
22. The method of Clause 17, wherein the sample is non-human in origin.
23. The method of Clause 17, wherein the sample is derived from an inanimate object.
24. The method of Clause 17, wherein the at least one forward primer, the at least one reverse primer and the one or more probes is selected from the group consisting of: Groups 1-152 of Table 2.
25. The method of Clause 17, further comprising adding an internal control plasmid and a positive control plasmid of Table 3 during detection of hybridization of the probe to the amplicon.
26. A kit for detecting and/or screening and/or quantitating CMV or EBV DNA in a sample, comprising one or more probes comprising a sequence selected from the group consisting of: SEQ ID NOS: 37-108 and 171.
27. The kit of Clause 26, farther comprising:
   a) at least one forward primer comprising a sequence selected from the group consisting of: SEQ ID NOS: 1-36, 176, 180, 182, 183, 186 and 191; and
   b) at least one reverse primer comprising a sequence selected from the group consisting of: SEQ ID NOS: 109-170, 172, 178, 181, 185, 188 and 190.
28. The kit of Clause 26, wherein the one or more probes are labeled with different detectable labels.
29. The kit of Clause 26, wherein the one or more probes are labeled with the same detectable label.
30. The kit of Clause 27, wherein the at least one forward primer, the at least one reverse primer and the one or more probes are selected from the group consisting of: Groups 1-152 of Table 2.
31. A method of diagnosing a CMV- or EB V-associated condition, syndrome or disease, comprising:
   a) contacting a sample with at least one forward and reverse primer set selected from the group consisting of: Groups 1-152 of Table 2;
   b) conducting an amplification reaction, thereby producing an amplicon; and
   c) detecting and/or screening and/or quantitating the amplicon using one or more probes selected from the group consisting of: SEQ ID NOS: 37-108,171, 177, 179, 184, 187 and 189, wherein the generation of an amplicon is indicative the presence of CMV or EBV in the sample.
32. The method of Clause 31, wherein the sample is selected from the group consisting of: blood; serum; plasma; enriched peripheral blood mononuclear cells; bone marrow; urine; neoplastic or other tissue obtained from biopsies; cerebrospinal fluid; saliva; fluids collected from the car, eye, mouth and respiratory airways; sputum; urine; stool; skin; semen; seminal fluid; gastric secretions; tears; oropharyngeal swabs; nasopharyngeal swabs; throat swabs; nasal aspirates; nasal wash; renal tissue and fluid therefrom including perfusion media; tissues either to be utilized for transplantation between individuals or animal-derived tissues to be utilized for transplantation into a human recipient; fluids and cells obtained by the perfusion of tissues of both human and animal origin; and fluids and cells derived from the cvltuhng of human cells, human stem cells, human cartilage or fibroblasts.
33. The method of Clause 31, wherein the CMV-associatcd condition, syndrome or disease is selected from the group consisting of: congenital, prenatal or perinatal CMV infection; CMV mononucleosis; post-transfusion CMV infection; solid organ transplantation CMV infection; hematopoietic stem cell transplantation CMV infection; CMV retinitis, pneumonitis; encephalitis; hepatitis; gastrointestinal disease; blindness; hearing ioss; and neurological disorders.
34. The method of clause 31, wherein the EBV-associated condition, syndrome or disease is selected from the group consisting of: lymphoproliferatjve disorders, neoplasia, myocarditis, encephalitis, pneumonia, mesenteric adenitis, hepatitis, EBV mononucleosis, nasopharyngeal carcinoma and pancreatitis.
35. A kit for amplifying and sequencing CMV and/or EBV DNA in a sample, comprising:
   a) at least one forward primer comprising the sequence selected from the group consisting of: SEQ ID NOS: 1-36, 176, 180, 182, 183,186 and 191 ;
   b) at least one reverse primer comprising the sequence selected from the group consisting of: SEQ ID NOS: 109-170, 172, 178, 181, 185, 188 and 190; and
   c) reagents for the sequencing of amplified DNA fragments utilizing standard sequencing chemistries.
36. A method of diagnosing a CMV- or EBV-associated condition, syndrome or disease, comprising contacting a denatured target from a sample with one or more probes selected from the group consisting of: SEQ ID NOS: 37-108, 171, 177, 179, 184, 187 and 189 for hybridization to occur; wherein hybridization of the probe to a denatured target is indicative of the presence of CMV or EBV in the sample.
37. The method of Clause 36, wherein the sample is selected from the group consisting of: blood; serum; plasma; enriched peripheral blood mononuclear cells; bone marrow; urine; neoplastic or other tissue obtained from biopsies; cerebrospinal fluid; saliva; fluids collected from the car, eye, mourn and respiratory airways; sputum; urine; stool; skin; semen; seminal fluid; gastric secretions; tears; oropharyngeal swabs; nasopharyngeal swabs; throat swabs; nasal aspirates; nasal wash; renal tissue and fluid therefrom including perfusion media; tissues either to be utilized for transplantation between individuals or animal-derived tissues to be utilized for transplantation into a human recipient; fluids and cells obtained by the perfusion of tissues of both human and animal origin; and fluids and cells derived from the culturing of human cells, human stem cells, human cartilage or fibroblasts.
38. The method of Clause 36, wherein the CMV-associated condition, syndrome or disease is selected from the group consisting of: congenital, prenatal or perinatal CMV infection; CMV mononucleosis; post-transfusion CMV infection; solid organ transplantation CMV infection; hematopoietic stem cell transplantation CMV infection; CMV retinitis, pneumonitis; encephalitis; hepatitis; gastrointestinal disease; blindness; hearing loss; and neurological disorders.
39. The method of clause 36, wherein the EBV-associated condition, syndrome or disease is selected from the group consisting of: lymphoproliferative disorders, neoplasia, myocarditis, encephalitis, pneumonia, mesenteric adenitis, hepatitis, EBV mononucleosis, nasopharyngeal carcinoma and pancreatitis.

## Claims

1. An isolated nucleic acid sequence comprising a sequence selected from the group consisting of: SEQ ID NOS: 186, 187 and 188.

2. A method of hybridizing one or more isolated nucleic acid sequences comprising an EBV target having a sequence selected from the group consisting of: SEQ ID NOS: 186, 187 and 188 to a EBV sequence, comprising contacting one or more isolated nucleic acid sequences to a sample comprising the EBV sequence under conditions suitable for hybridization.

3. A primer set comprising at least one forward primer comprising an EBV target sequence consisting essentially of the EBV target sequence of SEQ ID NO.186 and a reverse primer comprising an EBV target sequence consisting essentially of the EBV target sequence of SEQ ID NO. 188.

4. A probe comprising an EBV target sequence consisting essentially of the EBV target sequence of SEQ ID NO. 187.

5. A method for detecting and/or screening and/or quantitating EBV in a sample, comprising:
a) contacting the sample with a forward primer comprising an EBV target sequence consisting essentially of the EBV target sequence of SEQ ID NO. 186 and a reverse primer comprising an EBV target sequence consisting essentially of the EBV target sequence of SEQ ID NO. 188 under conditions such that nucleic acid amplification occurs to yield an amplicon; and
b) contacting the amplicon with a probe comprising an EBV target sequence consisting essentially of the EBV target sequence of SEQ ID NO. 187 under conditions such that hybridization of the probe to the amplicon occurs; wherein the hybridization of the probe is indicative of EBV in the sample, and wherein the extent of hybridization is optionally quantified.

6. A kit for detecting and/or screening and/or quantitating EBV DNA in a sample, comprising a probe comprising an EBV target sequence consisting essentially of the EBV target sequence of SEQ ID NO. 187.

7. The kit of Claim 6, further comprising:
a) a forward primer comprising an EBV target sequence consisting essentially of the EBV target sequence of SEQ ID NO. 186; and
b) a reverse primer comprising an EBV target sequence consisting essentially of the EBV target sequence of SEQ ID NOS: 188.

8. A method of diagnosing an EBV-associated condition, syndrome or disease, comprising contacting a denatured target from a sample with a probe comprising an EBV target sequence consisting essentially of the EBV target sequence of SEQ ID NO. 187 for hybridization to occur; wherein hybridization of the probe to a denatured target is indicative of the presence of EBV in the sample.

9. The method of Claim 8; wherein the sample is selected from the group consisting of: blood; serum; plasma; enriched peripheral blood mononuclear cells; bone marrow; urine; neoplastic or other tissue obtained from biopsies; cerebrospinal fluid; saliva; fluids collected from the car, eye, mourn and respiratory airways; sputum; urine; stool; skin; semen; seminal fluid; gastric secretions; tears; oropharyngeal swabs; nasopharyngeal swabs; throat swabs; nasal aspirates; nasal wash; renal tissue and fluid therefrom including perfusion media; tissues either to be utilized for transplantation between individuals or animal- derived tissues to be utilized for transplantation into a human recipient; fluids and cells obtained by the perfusion of tissues of both human and animal origin; and fluids and cells derived from the culturing of human cells, human stem cells, human cartilage or fibroblasts.

10. The method of Claim 8 or Claim 9, wherein the EBV-associated condition, syndrome or disease is selected from the group consisting of: lymphoproliferative disorders, neoplasia, myocarditis, encephalitis, pneumonia, mesenteric adenitis, hepatitis, EBV mononucleosis, nasopharyngeal carcinoma and pancreatitis.
